# EUROPEAN PATENT APPLICATION

(11) **EP 2 045 324 A1**
(43) Date of publication of application: **08.04.2009**
(21) Application number: 07791410.9
(22) Date of filing: 26.07.2007
(51) Int. Cl.: C12N 15/09, A61K 38/00, A61P 31/14, A61P 31/18, C12P 21/02

(54) **METHOD FOR PRODUCTION OF N36-BINDING PEPTIDE**

(30) Priority: 27.07.2006 JP 2006204827
(71) Applicant: Gekkeikan Sake Co., Ltd., Fushimi-ku Kyoto-shi Kyoto 6128660 (JP)
(72) Inventor: TSUTSUMI, Hiroko, Kyoto-shi Kyoto 612-8385 (JP); ISHIDA, Hiroki, Kyoto-shi Kyoto 612-8385 (JP); HISADA, Hiromoto, Kyoto-shi Kyoto 612-8385 (JP); MIZUMOTO, Makiko, Kyoto-shi Kyoto 612-8385 (JP); HATA, Yoji, Kyoto-shi Kyoto 612-8385 (JP); FUJII, Nobutaka, Kyoto-shi Kyoto 606-8501 (JP); MATSUOKA, Masao, Kyoto-shi Kyoto 606-8507 (JP); KODAMA, Eiichi, Kyoto-shi Kyoto 606-8507 (JP); OISHI, Shinya, Kyoto-shi Kyoto 606-8501 (JP)
(74) Representative: Müller-Boré & Partner Patentanwälte
(86) International application number: PCT/JP2007/064710
(87) International publication number: WO 2008/013242

(57) **Abstract**

The present invention relates to the production of an N36-binding peptide at low cost and in a large quantity utilizing a microorganism. More specifically, the present invention relates to a method for producing an N36-binding peptide comprising introducing a recombinant vector into which a DNA molecule encoding an N36-binding peptide that binds to an N36 protein derived from a retrovirus that causes immunodeficiency in a mammal has been incorporated into *E. coli* as a host to produce a transformant.

## Description

### TECHNICAL FIELD

The present invention relates to a method for producing, by utilizing a microorganism, an N36-binding peptide that has a preventive or therapeutic effect on retroviruses (e.g., HIV and SIV) that induce immunodeficiency in a mammal, and use of N36-binding peptide for preventing or treating diseases caused by AIDS-related viruses.

### BACKGROUND ART

AIDS is an abbreviation for Acquired Immune Deficiency Syndrome, and refers to a generic term for various diseases resulting from the malfunctioning immunity against pathogens caused by the infection of retroviruses (e.g., HIV and SIV) that induce immunodeficiency.

AIDS treatment agents used at present are reverse transcriptase inhibitors as represented by azidothymidine and inhibitors that block virus proliferation processes such as protease inhibitors, etc. Considerable therapeutic effects have been attained by combined therapy with these agents.

One of the known mechanisms by which a retrovirus, e.g., HIV, that causes immunodeficiency, enters a host cell is through C34 trimer with an α-helix structure in the gp41 protein of the HIV enveloping N36 trimer with an α-helix structure to form a hexamer, thereby causing the fusion of the HIV membrane with the host-cell membrane (Non-patent document 1). Drugs that target N36 for the prevention of the above hexamer formation, e.g., T20 (tradename "Fuzeon"), have been approved by the FDA of the Unite States; however, existence of a T20-resistant HIV-1 strain has already been confirmed. Therefore, a drug that has further anti-HIV activity is needed.

The C34 peptide, having stronger anti-HIV activity than T20, would hardly permit the emergence of drug-resistant viruses. However, it has extremely low water-solubility and the development thereof has been delayed. To improve the solubility of C34, the present inventors designed an N36-binding peptide so as to have an alpha-helix structure (Patent document 1). The N36-binding peptide has strong anti-HIV activity, and is also effective on the HIV strain that shows resistance to T20 drugs.
Since this N36-binding peptide is a designed peptide, the production thereof had to rely on chemical synthesis and the potential for production in large quantities has not been found. It is essential that the N36-binding peptide is produced in large quantities at low cost for practical use, since the amount required each year will reach about 1 ton and the cost of such synthesis is as much as 100 trillion yen.

If the N36-binding peptide can be produced by using a microorganism, it can be supplied practically as a therapeutic AIDS drug at low cost.

However, there are no preceding examples where the N36-binding peptide has been produced by using a microorganism.
Patent Document 1 WO03/029284
Non-patent Document 1 Review: D. M. Eckert, P. S. Kim, Annu. Rev. Biochem. 2001, 70, 777-810

### DISCLOSURE OF THE INVENTION

### PROBLEMS TO BE SOLVED BY THE INVENTION

An object of the present invention is to provide a method for producing an N36-binding peptide, which has been produced by chemical synthesis, at low cost and in large quantities by utilizing a microorganism.

### MEANS FOR SOLVING THE PROBLEMS

In view of the above prior art problems, the present inventors conducted extensive studies, and found that, in the production of a genetically engineered N36-binding peptide, the desired peptide can be produced at low cost in large quantities by utilizing at least one microorganism selected from the group consisting of *E*. *coli*, yeast and filamentous fungus as a host.

More specifically, the present invention provides the following methods for producing an N36-binding peptide, and a preventive or therapeutic agent composition for retrovirus infections that cause immunodeficiency in a mammal.
Item 1. A method for producing an N36-binding peptide, the method comprising introducing a recombinant vector incorporating a DNA molecule encoding the N36-binding peptide that binds to an N36 protein derived from a retrovirus that causes immunodeficiency in a mammal into *E. coli* as a host to produce a transformant, culturing the transformant in a medium to produce and accumulate the N36-binding peptide in the culture, and collecting the N36-binding peptide from the culture.
Item 2. The method according to Item 1, wherein said transformant is cultured in said medium, and the N36-binding peptide is produced and accumulated in the culture by intracellular expression, periplasm expression or secretion expression.
Item 3. The method according to Item 2, wherein said transformant is cultured in said medium and the N36-binding peptide is produced and accumulated by intracellular expression of *E. coli*.
Item 4. The method for producing an N36-binding peptide according to Item 1, wherein said recombinant vector contains the DNA molecule encoding an N36-binding peptide fused with a DNA molecule encoding a protein that can be expressed in *E. coli.*
Item 5. The method for producing an N36-binding peptide according to Item 1, wherein said recombinant vector contains the DNA molecule encoding an N36-binding peptide fused with a DNA molecule encoding a protein that can be expressed in *E. coli,* said DNA molecule encoding an N36-binding peptide containing at one end or both ends thereof a DNA molecule encoding a site capable of being recognized to be cleaved at a peptide bond.
Item 6. The method for producing an N36-binding peptide according to Item 5, wherein said recognition site is cysteine.
Item 7. The method for producing an N36-binding peptide according to Item 4, wherein said protein that can be expressed in *E. coli* is at least one member selected from the group consisting of glutathione S transferase and EGFP.
Item 8. A method for producing an N36-binding peptide, the method comprising introducing a recombinant vector incorporating a DNA molecule encoding an N36-binding peptide that binds to an N36 protein derived from a retrovirus that causes immunodeficiency in a mammal into a filamentous fungus as a host to produce a transformant, culturing the transformant in a medium to produce and accumulate the N36-binding peptide in the culture, and collecting the N36-binding peptide from the culture.
Item 9. The method for producing an N36-binding peptide according to Item 8, wherein said filamentous fungus is *Aspergillus oryzae*.
Item 10. The method for producing an N36-binding peptide according to Item 8 or 9, wherein said recombinant vector contains the DNA molecule encoding the N36-binding peptide fused with a DNA molecule encoding a protein that can be expressed in the filamentous fungus.
Item 11. The method for producing an N36-binding peptide according to Item 8, wherein said recombinant vector contains the DNA molecule encoding an N36-binding peptide fused with a DNA molecule encoding a protein that can be expressed in the filamentous fungus, said DNA molecule encoding the N36-binding peptide containing at one end or both ends thereof a DNA molecule encoding a site capable of being recognized to be cleaved at a peptide bond.
Item 12. The method for producing an N36-binding peptide according to Item 11, wherein said recognition site is cysteine.
Item 13. The method for producing an N36-binding peptide according to any one of Items 10 to 12, wherein said protein that can be expressed in the filamentous fungus is glucoamylase.
Item 14. The method for producing an N36-binding peptide according to any one of Items 8 to 13, wherein the filamentous fungus is cultured in a solid medium.
Item 15. A method for producing an N36-binding peptide, the method comprising introducing a recombinant vector incorporating a DNA molecule encoding an N36-binding peptide that binds to an N36 protein derived from a retrovirus that causes immunodeficiency in a mammal into a yeast as a host to produce a transformant, culturing the transformant in a medium to produce and accumulate the N36-binding peptide in the culture, and collecting the N36-binding peptide from the culture.
Item 16. The method for producing an N36-binding peptide according to Item 15, wherein said yeast is a microorganism of the genus Saccharomyces.
Item 17. The method for producing an N36-binding peptide according to Item 15 or 16, wherein said vector contains the DNA molecule encoding an N36-binding peptide and, fused thereto, a DNA molecule encoding a protein that can be expressed in the yeast.
Item 18. The method for producing an N36-binding peptide according to Item 15 or 16, wherein said recombinant vector contains the DNA molecule encoding an N36-binding peptide and, fused thereto, a DNA molecule encoding a protein that can be expressed in the yeast, said DNA molecule encoding an N36-binding peptide containing at one end or both ends thereof a DNA molecule encoding a site capable of being recognized to be cleaved at a peptide bond.
Item 19. The method for producing an N36-binding peptide according to Item 18, wherein said recognition site is cysteine.
Item 20. The method for producing an N36-binding peptide according to any one of Items 17 to 19, wherein said protein that can be expressed in the yeast is alpha-factor.
Item 21. A preventive or therapeutic agent composition for a retrovirus infection that causes immunodeficiency in a mammal, the composition containing as an effective component the N36-binding peptide obtained by the method of any one of Items 1 to 20.
Item 22. The preventive or therapeutic composition according to Item 21, wherein the retrovirus that causes immunodeficiency in a mammal is human immunodeficiency virus (HIV).

### EFFECTS OF THE INVENITON

According to the present invention, the N36-binding peptide can be produced in large quantities at low cost by utilizing a yeast, filamentous fungus, or *E. coli* as a host. Further, the present invention can provide a composition useful for preventing or treating diseases caused by a retrovirus that induces immunodeficiency in a mammal.

### BRIEF DESCRIPTION OF THE DRAWINGS

FIG. 1: construction of pSC34EK plasmid.
FIG. 2: construction of pGST-SC34EK-Histag.
FIG. 3: cultivation state of the transformed strains carrying respective plasmids
FIG. 4: SDS-PAGE analysis of the EGFP-SC34EK protein
FIG. 5: SDS-PAGE analysis of the GST-SC34EK-Histag protein
FIG. 6: SDS-PAGE analysis of the GST-SC34EK protein
FIG. 7: electrophoresis (bioanalyzer) of SC34EK (x 1)
FIG. 8: western blotting (SC34EK)
FIG. 9: western blotting (chromosome-integrated).
FIG. 10: expression by cultivating *Aspergillus oryzae* in liquid medium
FIG. 11: western analysis (liquid-state culture).
FIG. 12: SDS-PAGE analyses of solid-state culture
FIG. 13: purification of the fused protein expressed by solid-state cultivation
FIG. 14: ELISA assay (1. inhibitory reaction on N36-C34 binding, {Institute for Virus Research}, 2. assay performed by Institute for Virus Research)
FIG. 15: ELISA assay (3.)
FIG. 16: schematic diagram showing the Cys cleavage reactions

### BEST MODE FOR CARRYING OUT THE INVENTION

In the present invention, examples of retroviruses that cause immunodeficiency in a mammal include human immunodeficiency virus (HIV), simian immunodeficiency virus (SIV), bovine immunodeficiency-like virus, etc., with HIV being a representative example.

The "N36-bonding peptide" in the specification means the following.

### Definition of N36-binding Peptide

The polypeptide contains at least one modular structure of Modular Structures 1 to 3 below, and is capable of binding to N36 of the retrovirus that causes immunodeficiency in a mammal. The modular structure is a polypeptide consisting of 6 or 7 amino acids, and examples include the following 3 structures.
(Y') ₘ₁-X₁ₐX_{1b}-A₁-X_{1c}X_{1d}X₁ₑ-A₂-(X")ₙ₁: Modular Structure 1 (MS1)
(Y') ₘ₂-X₂ₐ-A₁A₁-X_{2b}X_{2c}-A₂A₂-(X")ₙ₂: Modular Structure 2 (MS2)
(Y') ₘ₃-X₃ₐ-A₁-X_{3b}X_{3c}X_{3d}-A₂-X': Modular Structure 3 (MS3)

In each module in the polypeptide, A₁ represents an acidic amino acid, preferably glutamic acid, aspartic acid or cysteic acid, and more preferably glutamic acid or aspartic acid. A₂ represents a basic amino acid, preferably lysine, arginine, ornithine, or histidine, more preferably lysine, arginine or ornithine, and further preferably lysine or ornithine.

In each module, A₁ and A₂ have the positional relationship of position i and position i+4. Such combination in which the amino acid at position i and the amino acid at position i+4 are an acidic amino acid and a basic amino acid, respectively (or a reverse combination thereof) leads to the formation of salt bridges between the amino acids, facilitating the formation of the α-helix structure of the peptide according to the present invention. Furthermore, the direction of the dipole of the salt bridge is opposite to the direction of the dipole formed by the peptide backbone, and therefore the thermodynamic stability is increased when the helix is formed.

In the combination of an acidic amino acid and a basic amino acid described above, the combination of glutamic acid (E) and lysine (K) is more preferable.

It is preferable that each module contain one or two combinations of an acidic amino acid and a basic amino acid described above.

Further, the N36-binding peptide may have the combination of position i and position i+4 described above not only within the module but also intermodularly (area beyond the module structures). The N36-binding peptide can have an even further stabilized α-helix structure by having such a combination between modules.

The amino acids X₁ₐ to X_{3d} (X₁ₐ, X_{1b}, X_{1c}, X_{1d}, X₁ₑ, X₂ₐ, X_{2b}, X_{2c}, X₃ₐ, X_{3b}, X_{3c}, and X_{3d}) in the N36-binding peptide represent the same or different amino acids.

Examples of such amino acids include glycine, alanine, valine, leucine, isoleucine, phenylalanine, tyrosine, tryptophan, serine, threonine, cysteine, cysteic acid, methionine, asparagine, glutamine, aspartic acid, glutamic acid, lysine, arginine, histidine, proline, ornithine, sarcosine, β-alanine, norleucine (Nle), naphthyl alanine (Nal), etc.

Proline should not be used when the α-helix structure is likely to be destroyed by the use thereof; however, it may be used when it is at an amino terminal (hereinafter referred to as "N-terminal") or a carboxyl terminal (hereinafter referred to as "C-terminal") and does not hence destroy α-helix structures.

Preferable examples of amino acids X₁ₐ, X₂ₐ or X₃ₐ include tryptophan, leucine, isoleucine, glutamine, serine, threonine, asparagine, valine, phenylalanine, tyrosine, methionine, glycine, alanine, naphthyl alanine, etc.

Examples of amino acid X_{1b} or the amino acid at position "c", namely, amino acid X_{3b} include glycine, alanine, valine, leucine, isoleucine, phenylalanine, tyrosine, tryptophan, serine, threonine, cysteine, cysteic acid, methionine, asparagine, glutamine, aspartic acid, glutamic acid, lysine, arginine, histidine, proline, ornithine, sarcosine, β-alanine, norleucine, naphthyl alanine, etc.

Proline should not be used when the α-helix structure may be destroyed by the use thereof; however, it may be used when it is at the N-terminal or C-terminal and does not hence destroy α-helix structures.

Preferable examples of amino acids X_{1c}, X_{2b}, X_{3c}, X₁ₐ, X_{2c} and X_{3d} include tryptophan, leucine, isoleucine, asparagine, glutamine, aspartic acid, glutamic acid, serine, threonine, valine, phenylalanine, tyrosine, methionine, glycine, alanine, etc.

Examples of amino acid X₁ₑ include glycine, alanine, valine, leucine, isoleucine, phenylalanine, tyrosine, tryptophan, serine, threonine, cysteine, cysteic acid, methionine, asparagine, glutamine, aspartic acid, glutamic acid, lysine, arginine, histidine, proline, ornithine, sarcosine, β-alanine, norleucine, naphthyl alanine, etc.

Proline should not be used when the α-helix structure may be destroyed by the use thereof; however, it may be used when it is at the N-terminal or C-terminal and, hence, does not destroy α-helix structures.

X' represents any amino acid -OR¹ or -NR²R³ that may have a protection group, and X" represents -OR⁴ or -NR⁵R⁶.

Examples of such amino acids include glycine, alanine, valine, leucine, isoleucine, phenylalanine, tyrosine, tryptophan, serine, threonine, cysteine, cysteic acid, methionine, asparagine, glutamine, aspartic acid, glutamic acid, lysine, arginine, histidine, proline, ornithine, sarcosine, β-alanine, norleucine, naphthyl alanine, etc.

Proline should not be used when the α-helix structure may be destroyed by the use thereof; however, it may be used when it is at the N-terminal or C-terminal and, hence, does not destroy α-helix structures.

When the N36-binding peptide has a protected amino acid, examples of the protection group include the following groups: An ethoxy carbonyl group, methoxy carbonyl group, 9-fluorenyl methoxy carbonyl group, benzyloxycarbonyl group, 4-methoxy benzyloxycarbonyl group, 2,2,2-trichloroethyloxy carbonyl group, formyl group, acetyl group, propionyl group, butyryl group, etc.

R¹ to R⁶ (R¹, R², R³, R⁴, R⁵, and R⁶), being the same or different, represent a hydrogen atom, an alkyl group, an aryl group or aralkyl group.

Examples of the alkyl group include C₁-C₄ linear- or branched-chain alkyl groups, and preferably include methyl, ethyl, propyl, isopropyl, butyl, isobutyl, tert-butyl, etc. Examples of the aryl group include a phenyl group and a phenyl group which has a substituent. Examples of a substituent include the alkyl group described above, a halogen atom, cyano, carboxylic acid, nitro, amino, acetylamino, alkoxy group, hydroxy group, etc. The number of substituents is 1 to 5, and preferably 1 to 3, but is not limited thereto.

Y' represents H, R⁷CO-, a toluene sulfonyl group, or a methane sulfonyl group. R⁷ represents an alkyl group, a phenyl group which may have a substituent, or a benzyl group which may have a substituent. Usable alkyl groups are those mentioned above. Examples and the number of substituents are also as mentioned above.

n1 is 1 when Module Structure 1 is at the C terminal, and n1 is 0 when Module Structure 1 is not at the C terminal. n2 is 1 when Module Structure 2 is at the C terminal, and is 0 when Module Structure 2 is not at the C terminal.

m1 is 1 when Module Structure 1 is at the N terminal, and is 0 when Module Structure 1 is not at the N terminal. m2 is 1 when Module Structure 2 is at the N-terminal, and is 0 when Module Structure 2 is not at the N-terminal. m3 is 1 when Module Structure 3 is at the N-terminal, and is 0 when Module Structure 3 is not at the N-terminal.

Further, in Module Structures 1 to 3, the amino acid refers to -NH-CH(R')-CO- (wherein R' represents a side chain of the amino acid.)

The polypeptide of the present invention is a polypeptide that contains one of the above Module Structures 1 to 3, and preferably more than one of Module Structures 1 to 3. The polypeptide of the present invention may consist of only module structures of the same modular structure.

The polypeptide of the present invention contains, for example, 2 to 15, preferably 3 to 12, and more preferably 4 to 10, of the above-mentioned module structures, with 5 to 7 being particularly preferable. The binding order of each module structure is not limited insofar as the N36-binding peptide forms an α-helix structure.

For example, one of the preferable aspects of the N36-binding peptide when used as an anti-HIV agent is the peptide that has Module Structure 1 at the amino terminal (hereinafter referred to as "N-terminal"). It is particularly preferable that Module Structure 1 present at the N-terminal be "Trp-Z-Glu-Trp-Asp-Arg-Lys" (wherein "Z" represents norleucine, methionine, or glutamic acid).

Another preferable embodiment is the peptide that has a Module Structure 3 at the C-terminal, and it is more preferable that X' be -NH₂.

In the N36-binding peptide, it is particularly preferable that no amino acids other than those constituting the module structures are present between each module. However, a 7-amio acids insertion sequence consisting of any 7 amino acids may be contained between each module, as long as the polypeptide of the present invention can form an α-helix structure and bind to N36. Any 1 to 6 amino acids may be added at the N-terminal or C-terminal.

Examples of arbitrary amino acids include glycine, alanine, valine, leucine, isoleucine, phenylalanine, thyrosin, tryptophan, serine, threonine, cysteine, cysteic acid, methionine, asparagine, glutamine, aspartic acid, glutamic acid, lysine, arginine, histidine, proline, ornithine, sarcosine, β-alanine, norleucine, naphthyl alanine, etc. These amino acids may optionally have a protection group as described above.

Proline should not be used when the α-helix structure may be destroyed by the use thereof; however, it may be used when it is at the N-terminal or C-terminal and does not hence destroy α-helix structures.

A preferable embodiment of a 7-amino acids insertion sequence that may be contained between each module is, for example, "X₂ₐ-A₂A₂-X_{2b}X_{2c}-A₁A₁" (X₂ₐ, X_{2b}, X_{2c}, A₁ and A₂ are defined as above).

The polypeptide of the present invention can contain one such 7-amino acids insertion sequence between module structures, and 1 to 3 at the N or C terminal.

Further, the N36-binding peptide may contain a compound capable of binding to N36 at its N and/or C terminals.

The N36-binding peptide can have, for example, 13 or 14 to 104 or 105, preferably 13 or 14 to 83 or 84, more preferably 13 or 14 to 48 or 49, and further preferably 34 or 35 amino acids.

The amino acid used in the N36-binding peptide is preferably L-form (L-amino acid), but D-form may also be used. When D-form is used, all optically active amino acids should be D-form.

Furthermore, the peptide bond (-NH-CO-⇔-N=C(OH)-) in the polypeptide of the present invention can be a biologically equivalent bond thereto, such as an alkene (-CH=CH-) or fluoroalkene (-CF=CH-). The alkene is described in, for example, S. Oishi, T. Kamano, A. Niida, Y. Odagaki, N. Hamanaka, M. Yamamoto, K. Ajito, H. Tamamura, A. Otaka and N. Fujii, "Diastereoselective Synthesis of New ψ[(E)-CH=CMe]- and ψ[(Z)-CH=CMe]-type Alkene Dipeptide Isosteres by Organocopper Reagents and Application to Conformationally Restricted Cyclic RGD Peptidomimetics", J. Org. Chem. 2002, 67, 6162-6173, etc.; and the fluoroalkene is described in, for example, A. Otaka, H. Watanabe, A. Yukimasa, S. Oishi, H. Tamamura, and N. Fujii, "New Access to a-Substituted (Z)-Fluoroalkene Dipeptide Isosteres Utilizing Organocopper Reagents under "Reduction-Oxidative Alkylation (R-OA)" Conditions, Tetrahedron Lett., 2001, 42, 5443-5446, etc.

Preferable embodiments of the N36-binding peptide are, for example, polypeptides represented by the following formulae, wherein MS1 represents Module Structure 1, MS2 represents Module Structure 2, MS3 represents Module Structure 3, and AA represents a 7-amino acid insertion sequence; MS1-MS1-MS1-MS1-MS1, MS2-MS2-MS2-MS2-MS2, MS3-MS3-MS3-MS3-MS3, AA-MS1-MS1-MS1-MS1, MS1-AA-MS1-MS1-MS1, MS1-MS1-AA-MS1-MS1,MS1-MS1-MS1-AA-MS1, MS1-MS1-MS1-MS1-AA, AA-MS2-MS2-MS2-MS2, MS2-AA-MS2-MS2-MS2, MS2-MS2-AA-MS2-MS2, MS2-MS2-MS2-AA-MS2, MS2-MS2-MS2-MS2-AA, MS3-AA-MS3-MS3-MS3, MS3-MS3-AA-MS3-MS3, MS3-MS3-MS3-AA-MS3, MS3-MS3-MS3-MS3-AA, AA-MS3-MS3-MS3-MS3, MS1-MS2-AA-MS1-MS3, MS1-MS2-MS2-MS1-MS3, MS1-AA-MS2-MS1-MS3, MS1-MS2-MS1-AA-MS3, MS1-MS2-MS2-MS1-MS3, MS1-MS1-MS2-MS1-MS3, MS1-MS1-MS2-MS2-MS3, MS1-MS1-MS2-MS3-MS3, MS1-MS1-MS1-MS1-MS3, MS1-MS1-MS1-MS2-MS3, MS1-MS1-MS1-MS3-MS3, MS1-MS1-MS3-MS1-MS3, MS1-MS1-MS3-MS2-MS3, MS1-MS1-MS3-MS3-MS3, MS1-MS2-MS1-MS1-MS3, MS1-MS2-MS1-MS2-MS3, MS1-MS2-MS1-MS3-MS3, MS1-MS2-MS2-MS2-MS3, MS1-MS2-MS2-MS3-MS3, MS1-MS2-MS3-MS1-MS3, MS1-MS2-MS3-MS2-MS3, MS1-MS2-MS3-MS3-MS3, MS1-MS3-MS1-MS1-MS3, MS1-MS3-MS1-MS2-MS3, MS1-MS3-MS1-MS3-MS3, MS1-MS3-MS2-MS1-MS3, MS1-MS3-MS2-MS2-MS3, MS1-MS3-MS2-MS3-MS3, MS1-MS3-MS3-MS1-MS3, MS1-MS3-MS3-MS2-MS3, and MS1-MS3-MS3-MS3-MS3.

Specific examples of further preferable N36 proteins include "Ac-Trp-Nle-Glu-Trp-Asp-Arg-Lys-Ile-Glu-Glu-Tyr-Thr-Lys-Lys-Ile-Lys-Lys-Leu-Ile-Glu-Glu-Ser-Gln-Glu-Gln-Gln-Glu-Lys-Asn-Glu-Lys-Glu-Leu-Lys-NH2" (SC34-a), "Ac-Trp-Met-Glu-Trp-Asp-Arg-Lys-Ile-Glu-Glu-Tyr-Thr-Lys-Lys-Ile-Lys-Lys-Leu-Ile-Glu-Glu-Ser-Gln-Glu-Gln-Gln-Glu-Lys-Asn-Glu-Lys-Glu-Leu-Lys-NH2" (SC34-b), "Ac-Trp-Nle-Glu-Trp-Asp-Arg-Lys-Ile-Glu-Glu-Tyr-Thr-Lys-Lys-Ile-Glu-Glu-Leu-Ile-Lys-Lys-Ser-Gln-Glu-Gln-Gln-Glu-Lys-Asn-Glu-Lys-Glu-Leu-Lys-NH2" (SC34(EK)-a), "Ac-Trp-Met-Glu-Trp-Asp-Arg-Lys-Ile-Glu-Glu-Tyr-Thr-Lys-Lys-Ile-Glu-Glu-Leu-Ile-Lys-Lys-Ser-Gln-Glu-Gln-Gln-Glu-Lys-Asn-Glu-Lys-Glu-Leu-Lys-NH2" (SC34(EK)-b), "Ac-Trp-Glu-Glu-Trp-Asp-Lys-Lys-Ile-Glu-Glu-Tyr-Thr-Lys-Lys-Ile-Glu-Glu-Leu-Ile-Lys-Lys-Ser-Glu-Glu-Gln-Gln-Lys-Lys-Asn-Glu-Glu-Glu-Leu-Lys-Lys-NH2" (SC35 (EK)) (wherein "Ac" represents an acetyl group), etc.

The above examples of the N36 protein are sequences mainly effective for HIV, and amino acid sequences of the N36 protein for simian immunodeficiency virus (SIV) and bovine immunodeficiency-like virus (BIV) can be designed in the same manner in accordance with the corresponding C34 peptide sequence of a retrovirus that causes immunodeficiency.

The N36 protein is a protein that has an α-helix structure and to which a C34 trimer, having an α-helix structure present in the gp41 protein of the retrovirus that causes immunodeficiency in a mammal, is bound.

The N36-binding peptide includes C34 and derivatives thereof. C34 derivatives capable of binding to N36 include those having amino acid residues substituted, extended, or deleted for the purpose of improving the water-solubility and stability of C34. Examples of C34 derivatives are, as disclosed in Unexamined Japanese Patent Publication No. 2003-176298, those having plural module structures, each consisting of 6 or 7 amino acids, wherein the amino acid at position i and the amino acid at position i+4 are in a combination of an acidic amino acid and a basic amino acid (or may be in the reverse combination), whereby a salt bridge formed between these amino acids and α-helix structure of the polypeptide of the present invention is easily conformed. In the specification, derivatives containing a single module (X-EE-XX-KK), wherein the amino acid at position i and the amino acid at position i+4 of the C34 protein are substituted with an acidic amino acid (indicated as "E" as representing Glu (E)) and a basic amino acid (indicated as "K" as representing Lys(K)), respectively, are sometimes referred to as "SC34" derivatives, and derivatives containing two or more of such a module are sometimes referred to as "SC34EK" derivatives. The N36-binding peptide encompases those having a carboxyl group or an amino group at its terminal and those having other chemical structures (amido group, lactam ring, etc.) substituted when the N36-binding peptide is cleaved from a fused protein. Further, the peptide terminal of the obtained fused protein-peptide may be substituted with other chemical structures by a common method. Furthermore, the peptide of the present invention can be used, in addition to the form of a peptide, in the form of a derivative wherein the peptide bond is substituted with a structure such as an ether bond, a carbon bond, e.g., alkene, fluoroalkene, alkane, etc. Due to the substitution of the peptide bond, the peptide is hardly decomposed by enzymes, whereby an improvement in stability in vivo can be expected.

The amino sequence encoding the N36-binding peptide of HIV used in the examples of the present invention is shown under SEQ ID NO: 1 (Trp-Met-Glu-Trp-Asp-Arg-Lys-Ile-Glu-Glu-Tyr-Thr-Lys-Lys-Ile-Glu-Glu-Leu-Ile-Lys-Lys-Ser-Gln-Glu-Gln-Gln-Glu-Lys-Asn-Glu-Lys-Glu-Leu-Lys), and the base sequence is shown under SEQ ID NO: 2.

The host cells used in the production method of the present invention are, for example, *E. coli*, filemantous fungi, and yeasts. Examples of *E. coli* include those belonging to *Escherichia coli*, and those having a parent strain of either B strain or K12 strain can also be used. Specific examples of *E. coli* include, but are not limited thereto, commercial XLl-Blue strain, BL-21 strain, JM107 strain, TB1 strain, JM109 strain, C600 strain, DH5α strain, HB101 strain, etc. Examples of filamentous fungi include, but are not limited thereto, various *Aspergillus koji* (sake koji, miso koji, soy sauce koji, mirin koji, distilled liquor koji, etc.), those belonging to genus *Acremonium*, genus *Humicola*, genus *Aspergillus,* genus *Trichoderma*, genus *Fusarium*, genus *Penicillium*, genus *Mucor*, genus *Rhizopus*, genus *Neurospora*, etc. Among these, production using genus *Aspergillus* is preferable. Specific examples include, but are not limited thereto, *Aspergillus niger, Aspergillus oryzae, Aspergillus awamori, Aspergillus kawachii, Aspergillus usamii, Aspergillus sojae, Aspergillus fumigatus, Aspergillus japonicus, Aspergillus flavus, Aspergillus nidulans, Aspergillus aculeatus, Aspergillus terreus, Aspergillus parasiticus, Aspergillus saitoi,* etc. Examples of yeast include, but are not limited thereto, those of genus *Saccharomyces*, genus *Hansenula*, genus *Pichia*, genus *Schizosaccharomyces*, and genus *Kluyveromyces*. Specific examples include *Saccharomyces cerevisiae, Pichia pastoris, Pichia methanolica, Schizosaccharomyces pombe, Hansenula anomala, Kluyveromyces lactis*, etc.

When the host is *E. coli,* it is more preferable to use, for example, the promoter region of an inducible enzyme as promoter. Specific examples of preferable promoters include trp promoter, lac promoter, recA promoter, lpp promoter, tac promoter, T7 promoter, λPL promoter, etc. When the host is a yeast, preferable examples include PHO5 promoter, PGK promoter, GAP promoter, ADH1 promoter, GAL promoter, etc. When the host is a filamentous fungus, usable promoters include all promoters capable of functioning in a filamentous fungus, and encompass those in which a sequence modification is incorporated into these promoters. Usable promoters include any of glycolysis-related genes, genes related to constitutive expression, and hydrolysis-related enzyme genes. Specific examples of preferable genes include, but are not limited thereto, amyB glaA, agdA, glaB, TEF1, xynF1tannase gene, No.8AN, gpdA, pgkA, enoA, melO, sodM, catA, catB, etc.

When the N36-binding peptide is expressed singly in a host, the yield of the intended peptide is low even using various high expression production systems. For this reason, it is preferable that the N36-binding peptide be linked to a polypeptide that can be expressed in a host, or the N36-binding peptide be expressed as a multimer (e.g., trimer to heptamer). Further, to easily collect the intended N36-binding peptide after expression, it is preferable that the N36-binding peptide be linked to (fused with) a polypeptide that can be expressed in a host wherein the peptide bond is mediated by a cleavable recognition site.

A preferable polypeptide fused with the N36-binding peptide is not limited insofar as it can be expressed in a host. When the host is *E. coli*, examples include DsRed, GFP, EGFP, etc., that can be visualized, and more preferably MBP, glutathione S transferase (GST), T7tag, Trxtag, Stag, CBDtag, Nustag, etc., that can be affinity-purified. When the host is a yeast, examples of the polypeptide include α-factor, invertase, acid phosphatase, various types of secretion protein-glucoamylase, α-amylase, etc. When the host is a filamentous fungus, preferable examples of the polypeptide include pectin decomposition enzymes such as phytase, lyase, pectinase, etc.; proteolytic enzymes such as amylase, glucoamylase, α-galactosidase, β-galactosidase, α-glucosidase, β-glucosidase, mannosidase, isomerase, invertase, transferase, ribonuclease, deoxyribonuclease, chitinase, catalase, laccase, phenol oxidase, oxidase, oxidoreductase, cellulase, xylanase, peroxidase, lipase, hydrolase, esterase, cutinase, protease, etc.; and protein structure genes such as aminopeptidase or carboxypeptidase, etc. Such a polypeptide that can be expressed in a host may have the entire sequence or may have a partial sequence as long as it enables the expression of the N36-binding peptide. Further, it is desirable that such a polypeptide be derived from a host, but it may be derived from a species other than a host cell or modifications thereof, insofar as it can be expressed in a host.

The recognition site mentioned earlier encompasses all sites capable of cleaving fused peptides, and examples include amino acid sequences (consisting of two or more amino acids) that can be cleaved by a protease such as processing enzyme or digestive enzyme; amino acids that can be cleaved by physical cleavage using chemical modification agents such as cysteine, methionine, etc., ultrasound, laser, heat, or the like. Examples of a digestive enzyme (processing enzyme) that can cleave a recognition site preferably include Factor Xa, thrombin, renin, trypsin, V8 protease, pseudomonas endoprotease, Arthrobacter lysyl endopeptidase, etc. The recognition site is, for example, an Ile-Glu-Gly-Arg sequence that recognizes Factor Xa. Examples of chemical modification agents include CNBr, Asn, and Asp, that recognize Met; dilute hydrochloric acid that recognizes Glu; and DMAP-CN that recognizes cysteine. For example, when cysteine residues are arranged at the linkage site of the N36-binding peptide and a polypeptide, the cleavage can be carried out by the S-cyano method using DMAP-CN.

The N36-binding peptide may be accumulated within a host, or secreted outside a host (into a culture solution or periplasm when the host is *E. coli*).

In the present invention, the fused (chimeric) protein of the N36-binding peptide and a protein that can be expressed in a host can be obtained by transforming a host, such as *E. coli*, yeast, filamentous fungus, with a recombinant vector containing a chimeric DNA to which DNA encoding these peptides and proteins are directly connected in-frame, or, as necessary, together with DNA encoding a recognition site.

DNA encoding a polypeptide to be fused with the N36-binding peptide can be cloned by synthesizing a suitable pair of oligonucleotide primers based on a known gene sequence, and performing RT-PCR or PCR using as a template the entire RNA or polyA⁽⁺⁾RNA extracted from the host cell or tissue of the gene, or using chromosomal DNA. During this process, to facilitate the subsequent cloning into a vector, a suitable sequence for recognizing a restriction enzyme can be added at the terminals of the oligo primers used.

In the present invention, DNA encoding for the N36-binding peptide can be obtained by determining a base sequence based on its amino acid sequence in consideration of the codon usage in a host, synthesizing a partial sequence of the sense strand and a partial sequence of the antisense strand in such a manner that they partially overlap using an automatic DNA/RNA synthesizer, and repeating an operation by which longer partial sequences are obtained as double-stranded DNAs by PCR.

When the host is *E. coli*, the polypeptide fused with the N36-binding peptide can also have a signal peptide, and may be a protein that is secreted in the periplasm or outside a cell.

The recombinant vector used in the present invention can be any recombinant vector wherein DNA, coding for the N36-binding peptide or a fused (chimeric) protein of the N36-binding peptide and a protein that can be expressed in a host, is present under the control of a promoter that is capable of functioning in a host such as *E. coli*, yeast or filamentous fungus. The promoter region typically contains consensus sequences, the -35 region and the -10 region, that determine the binding site of RNA polymerase, however, it may contain other sequences that can determine the binding site of RNA polymerase. It is more desirable to use the promoter region of an inducible enzyme as a system for large expression of the desired recombinant protein. When an inducer (e.g., lactose or IPTG for lac promoter) is added, the binding of a repressor protein to an operator is inhibited, whereby the DNA under the control of the promoter is expressed in large quantities. The recombinant vector contains a Shine-Dalgarno (SD) sequence upstream of the translation initiation codon. The recombinant vector further contains a transcription termination signal, i.e., a termination region, downstream of the DNA encoding the N36-binding peptide or a fused (chimeric) protein of the N36-binding protein and a protein that can be expressed in a host. Usable terminator regions include natural or synthesized terminators that are commonly employed. The recombinant vector of the present invention also contains the replication origin for autonomous replication in a host, in addition to the above-mentioned promoter region and terminator region.

It is preferable that the recombinant vector of the present invention further contain a selection marker gene for selecting a transformant. Examples of selection marker genes for *E. coli* include, but are not limited thereto, various genes tolerant to drugs such as tetracycline, ampicillin, kanamycin, etc., and recessive selection markers complementary to mutations of a gene involved in auxotrophy can also be used. Examples of selection marker genes for yeast include, but are not limited thereto, genes tolerant to geneticin; genes complementary to mutations of a gene involved in auxotrophy; and selection markers such as LEU2, URA3, TRP1, HIS3, etc. Examples of selection marker genes for filamentous fungus include, but are not limited thereto, those selected from the group consisting of niaD (Biosci. Biotechnol. Biochem., 59, 1795-1797, 1995), argB (Enzyme Microbiol Technol, 6, 386-389, 1984), sC (Gene, 84, 329-334, 1989), ptrA (Biosci Biotechnol Biochem, 64, 1416-1421, 2000), pyrG (Biochem Biophys Res Commun, 112, 284-289, 1983), amdS (Gene, 26, 205-221, 1983), Aureobasidin-resistant gene (Mol Gen Genet, 261, 290-296, 1999), benomyl-tolerant gene (Proc Natl Acad Sci USA, 83, 4869-4873, 1986) and hygromycin-tolerant gene (Gene, 57, 21-26, 1987); leucine auxotroph complementary gene, etc. Further, when a host is an auxotrophic mutant strain, a wild-type gene complementary to the auxotrophy can also be used as a selection marker gene.

The introduction of the recombinant vector into a host cell can be carried out by a known method. Examples include methods such as the calcium chloride method by Cohen et al., disclosed in Proc. Natl. Acad. Sci. USA, 69:2110, 1972; the protoplast method in Mol. Gen. Genet., 168:111, 1979; the competent method in J. Mol. Biol., 56:209, 1971; electropolation, etc.

The N36-binding peptide or protein that can be expressed with the N36-binding peptide in a host can be obtained by culturing the above transformant in a suitable medium, and isolating it from the obtained culture.

Usable media are those containing carbohydrate as a carbon source such as glucose, fructose, glycerol, starch, etc. Usable media may further contain an inorganic or organic nitrogen source such as ammonium sulfate, ammonium chloride, hydrolyzed casein, yeast extract, polypeptone, bacto tryptone, beef extract, etc. These carbon sources and nitrogen sources do not have to be used in the pure form. Those of a low purity are also advantageous because they contain a small amount of growth factor and a large amount of inorganic nutrient. Further, if desired, the medium may contain other nutrient sources, such as mineral salts, e.g., sodium diphosphate or potassium diphosphate, dipotassium hydrogenphosphate, magnesium chloride, magnesium sulfate, calcium chloride; vitamins, e.g., vitamin B1, etc.; antibiotics, e.g., ampicillin, kanamycin, etc.

The transformant is cultured at a pH of typically 5.5 to 8.5, and preferably 6 to 8, at typically 18 to 40°C, preferably 20 to 35°C, for 1 to 150 hours, and these conditions can be suitably changed according to culture conditions and culture scale.

When the culture is carried out in a large tank, to avoid growth retardation of the intended protein during the production process, it is preferable that cells be inoculated in a small amount of medium for about 1 to 24 hours, and the obtained culture be subsequently inoculated in a large tank.

When the intended protein is controlled by the promoter system of an inductive protein gene, an inducing substance may be added at the initiation of the culture, but is preferably added at the beginning of the logarithmic phase. The cell growth of a host can be monitered by measuring an absorbance of the culture solution at 660 nm. For example, when lac promoter and tac promoter are used, isopropylthio-β-D-galactoside (hereinafter sometimes abbreviated to IPTG), as an inducing substance, can be added so as to be 0.1 to 1.0 mM when the absorbance reaches 0.4 to 0.8 at 660 nm. The addition time and addition rate of an inducing substance can be suitably changed in accordance with culture conditions, culture scale, type of inducing substance, etc.

According to the present invention, the N36-binding peptide or chimeric protein thereof is purified by binding a tag or label, such as Histag, etc., to the peptide or protein. Alternatively, the purification can be performed by affinity chromatography wherein a protein such as GST, MBP (maltose-binding protein), or the like, is bound to the peptide or protein. The purification may further be performed by a common purification operation such as gel filtration, electrophoresis, isoelectric chromatography, etc.

Subsequently, the chimeric protein wherein the N36-binding peptide is bound to Histag, or to a protein such as GST, MBP, T7tag, Trxtag, Stag, CBDtag, Stag, or Nustag (pET system manual, Merck Publication), or the like, has its label such as Histag and/or a protein such as GST, MBP, T7tag, Trxtag, Stag, CBDtag, Stag, Nustag, or the like, cleaved at a specific protease (e.g., thrombin, enterokinase, etc.) recognition site, whereby the intended N36-binding peptide is obtained. The recognition site is designed by sequencing amino acids by genetic engineering, and various sequence-specific restriction enzymes (particularly protease) can be caused to react with the recognition site. Alternatively, when an amino acid such as cysteine is used as a recognition site, the recognition site is cleaved using a chemical modification agent such as DMAP-CN, etc., to obtain the desired N36-binding peptide. When the desired protein is secreted in the periplasm of *E. coli*, cells are spheroplasted by lysozyme treatment, or the like, the desired protein is isolated in a solution, the cells are removed by filtration or centrifugal separation, and the obtained supernatant is subjected to a purification method such as affinity chromatography, etc. When the desired proteins are secreted outside *E. coli*, a purification method such as affinity chromatography can also be used.

When the desired protein is present within a host, the host cells are collected from the culture, a fraction containing the desired protein is obtained by cell disruption, or the like, and the obtained fraction is purified by a technique such as affinity chromatography.

The AIDS preventive or therapeutic agent for a mammal containing an active component that can be produced by the method of the present invention is not limited insofar as it contains the N36-binding peptide. The AIDS preventive or therapeutic agent for a mammal of the present invention can optionally contain in accordance with the formation of use, a biologically acceptable carrier, excipient, etc. The AIDS preventive or therapeutic agent for a mammal of the present invention can be produced by a common method. For example, the agent can be used orally in the form of tablets that are sugar coated or enteric coated as necessary, capsules, elixirs, microcapsules, etc.; or parenterally in the form of external preparations such as ointments, plasters, etc.; percutaneously, nasotracheally or transtracheally in the form of nebulas, inhalants, etc.; or in the form of injectable solutions such as suspension agents or an aseptic solution of water and other pharmaceutically acceptable liquids, etc.

The dose of the AIDS preventive or therapeutic agent of the present invention varies depending on symptoms, etc., but can be generally effective, when administered orally, in an amount of about 100 to about 4000 mg, preferably about 200 to 3000 mg, and more preferably about 300 to 2000 mg, a day per adult having a body weight of 60 kg. When administered parenterally, a single dose of the agent varies depending on the subject, the organ to be treated, symptons, administration route, etc., and, for example, for an intravenous administration in the form of an injection to an adult transplant patient having a body weight of 60 kg, it is favorable that the agent is intravenously administered in the form of an injection in a dose of about 0.00001 to about 1 g, preferably about 0.01 to about 30 mg, more preferably about 0.1 to about 20 mg, and further preferably about 0.1 to about 10 mg. This dose is also effective for other mammals.

### EXAMPLES

### Examples

The following will describe the present invention in more detail based on examples. It should be noted, however, that the invention is in no way limited by the descriptions below.

### Example 1: Production of SC34EK Using Escherichia coli as a Host E. coli Host

### E. coli BL21(DE3) strain (Merck) was used as the host for genetic transformation.

### Construction of Protein and Peptide Expression Plasmids

### (i) Plasmid Construction

Plasmids were constructed according to the following procedure. First, the PCR amplification products were subjected to phenol-chloroform extraction followed by ethanol precipitation. The products were then treated with restriction enzymes corresponding to the restriction enzyme recognition sites incorporated in the primers. The resulting gene fragments were separated by agarose gel electrophoresis, and extracted with a QIAquick Gel Extraction kit (Qiagen). Then, the products were mixed with gene fragments of plasmids treated with the corresponding restriction enzymes, and allowed to react at 16°C for 20 hours to ligate, using a Ligation kit ver. 2.1 (Takara Bio Inc.). The reaction mixture was transformed into *E. coli* JM109 competent cells (Takara Bio Inc.), and plasmids were extracted after incubation. By restriction enzyme mapping and checking the base sequence, a plasmid matching the required criteria was selected.

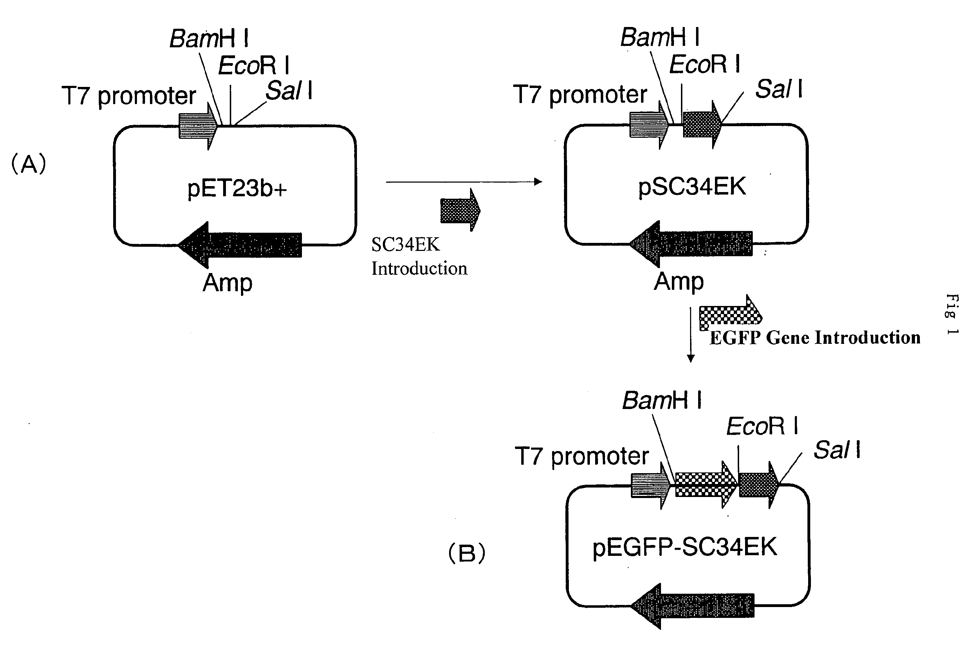

### (ii) pSC34EK Plasmid Construction (Fig. 1)

pET23b+, pET41b+, and pLysS (all available from Merck) were used as protein expression plasmids. The gene fragment (SEQ ID NO: 2) encoding a Cys-SC34EK-Cys peptide was synthesized by PCR using the polynucleotides represented by SEQ ID NO: 3 and SEQ ID NO: 4. All PCR reactions were performed with the included enzyme, buffer, and substrate of LA-Taq (Takara Bio Inc.).

### PCR Conditions

· 96°C (1 min), 1 cycle
· 96°C (20 sec), 60°C (30 sec), 72°C (30 sec), 5 cycles
· 72°C (2 min), 1 cycle

Next, to incorporate the restriction enzyme recognition sites in the gene fragment (SEQ ID NO: 2), a PCR reaction was performed using the gene fragment (SEQ ID NO: 2), prepared with SEQ ID NO: 3 and 4, as a template. As the primers, SEQ ID NO: 5 (CCCGGAATTCGAGCCTCGAGTGCTGGATGGAATGGGATCGC, EcoRI recognition site underlined) and SEQ ID NO: 6 (ACGCGTCGACGCATTTCAGTTCTTTTTCG, SalI recognition site underlined) were used.

### PCR Conditions

· 96°C (1 min), 1 cycle
· 96°C (20 sec), 60°C (30 sec), 72°C (30 sec), 30 cycles
· 72°C (2 min), 1 cycle

The resulting Gene Fragment I was digested with restriction enzymes EcoRI and SalI, and introduced into the corresponding recognition sites in the multiple cloning site of the pET23b+ plasmid, so as to prepare a pSC34EK plasmid capable of expressing a chimeric protein fusing Cys-SC34EK-Cys and Histag.

### (ii) pEGFP-SC34EK Plasmid Construction

A PCR reaction was performed using an EGFP gene fragment (Biochem Biophys Res Commun. 1996 Oct 23; 227(3):707-11) as a template. A primer pair represented by SEQ ID NO: 7 (CCGCGGATCCGATGGTGAGCAAGGGCGAGG, BamHI recognition site underlined) and SEQ ID NO: 8 (CCCGGAATTCGGCTTGTACAGCTCGTCCAT, EcoRI recognition site underlined) was used for the reaction.

### PCR Conditions

· 96°C (1 min), 1 cycle
· 96°C (20 sec), 60°C (30 sec), 72°C (1 min), 30 cycles
· 72°C (2 min), 1 cycle

The resulting EGFP gene fragment was digested with restriction enzymes BamHI and EcoRI, and introduced into the corresponding recognition sites in the multiple cloning site of the pSC34EK plasmid, so as to prepare a pEGFP-SC34EK plasmid capable of expressing a chimeric protein fusing EGFP, Cys-SC34EK-Cys, and Histag in this order (Fig. 1).

### (iii) pGST-SC34EK-Histag Plasmid Construction

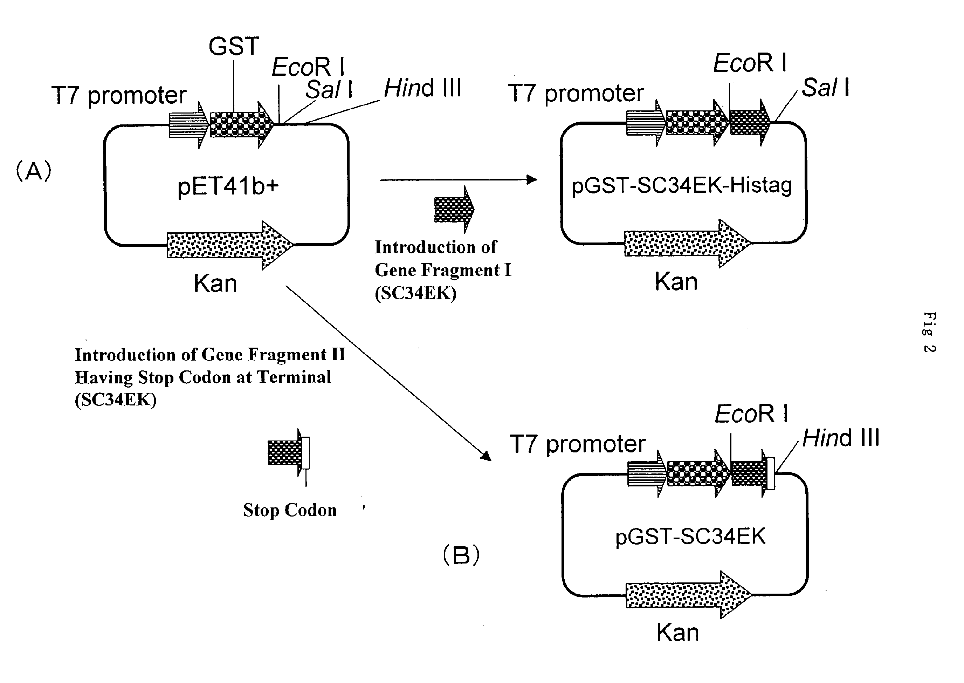

The Gene Fragment I was digested with restriction enzymes EcoRI and SalI, and introduced into the corresponding recognition sites in the multiple cloning site of the pET41b+ plasmid, so as to prepare a pGST-SC34EK-Histag plasmid capable of encoding a chimeric protein fusing GST, Cys-SC34EK-Cys, and Histag in this order (Fig. 2).

### (iv) pGST-SC34EK Plasmid Construction

The terminal Cys residue of the gene fragment encoding the Cys-SC34EK-Cys peptide was modified to a stop codon by PCR reaction, using the gene fragment (SEQ ID NO: 2) as a template. As the primers, SEQ ID NO: 5 and SEQ ID NO: 9 (TTTTTAAGCTTTCATTTCAGTTCTTTTTCGTTT, HindIII recognition site underlined) were used.

### PCR Conditions

· 96°C (1 min), 1 cycle
· 96°C (20 sec), 60°C (30 sec), 72°C (30 sec), 30 cycles
· 72°C (2 min), 1 cycle

The resulting Gene Fragment II was digested with restriction enzymes EcoRI and HindIII, and introduced into the corresponding recognition sites in the multiple cloning site of the pET41b+ plasmid, so as to prepare a pGST-SC34EK plasmid encoding a chimeric protein fusing GST and Cys-SC34EK (Fig. 2).

### Protein Expression

### Selection of Transformants by Chemicals

Selection of the host *E. coli* BL21 (DE3) strain transformed with pET23b+, pSC34EK, and pEGFP-SC34EK plasmids was made using the antibiotic ampicillin in agar medium and liquid medium. Selection of the host *E. coli* BL21(DE3) strain transformed with pET41b+, pGST-SC34EK-Histag, and pGST-SC34EK plasmids was made using the antibiotic kanamycin in agar medium and liquid medium. When the pLysS plasmid was further introduced into the host, selection was made by supplementing the media with the antibiotic chloramphenicol.

### Incubation of E. coli and Protein Expression

The plasmids were introduced into *E. coli* BL21 (DE3) strain competent cells (Merck) to transform the cells. After the reaction, the competent cells were plated on antibiotic-supplemented LB agar medium, and incubated for 8 hours at 37°C to form colonies. The colonies were gently picked with a toothpick sterilized by autoclaving, and inoculated in LB liquid medium supplemented with antibiotic. The cells were shake cultured for 8 hours at 37°C to prepare a preculture, which was then added to 100 ml to 2,000 ml of antibiotic-supplemented LB liquid medium in an amount 1/50 of the medium. This was followed by main culturing by shaking for 5 hours at 37°C. In the main culture, absorbance (ABS660) was measured at 1-hour intervals to quantify cell concentrations. First, after 2 hours of shake culturing at 37°C, IPTG was added (0.1 to 1.0 mM) to activate the T7 promoter in the plasmids and induce protein expression. *E. coli* was collected after further shake culturing for 3 hours at 37°C and subsequent centrifugation. The cells were then suspended in 2 to 10 ml of 10 mM phosphate sodium buffer (pH 6 to 8), and disrupted by sonication or high pressure. After recentrifugation, the supernatant was collected to obtain a supernatant sample.

### Protein Purification

For purification using Histag, a nickel column (Amersham Pharmacia) was used. First, the nickel column was equilibrated with 10 to 100 mM phosphate buffer (pH 7.4 to 7.6), and the supernatant sample was adsorbed on the column after adjusting the protein concentration to 2 to 20 mg/ml. Then, using 10 to 100 mM phosphate buffer (pH 7.4 to 7.6) and a mixed buffer of 0.1 to 1.0 M NaCl and 10 to 200 mM imidazole, contaminant proteins were eluted from the column. Target proteins were eluted with 10 to 100 mM phosphate buffer (pH 7.4 to 7.6) and 300 to 1000 mM imidazole buffer. The eluate was used as a purified sample.

For purification using GST-tag, a glutathione column (Pierce) was used. First, the glutathione column was equilibrated with 10 to 100 mM phosphate buffer (pH 6.0 to 8.0), and the supernatant sample was adsorbed on the column after adjusting the protein concentration to 2 to 20 mg/ml. Then, using 10 to 100 mM phosphate buffer (pH 7.4 to 7.6) and a mixed buffer of 0.1 to 1.0 M NaCl and 0.1 to 1 mM reduced glutathione, contaminant proteins were eluted from the column. Target proteins were eluted with 10 to 100 mM Tris buffer (pH 6.0 to 8.0) and 1 to 20 mM reduced glutathione buffer. The eluate was used as a purified sample.

### Culture Conditions of Transformed Strains with Plasmids

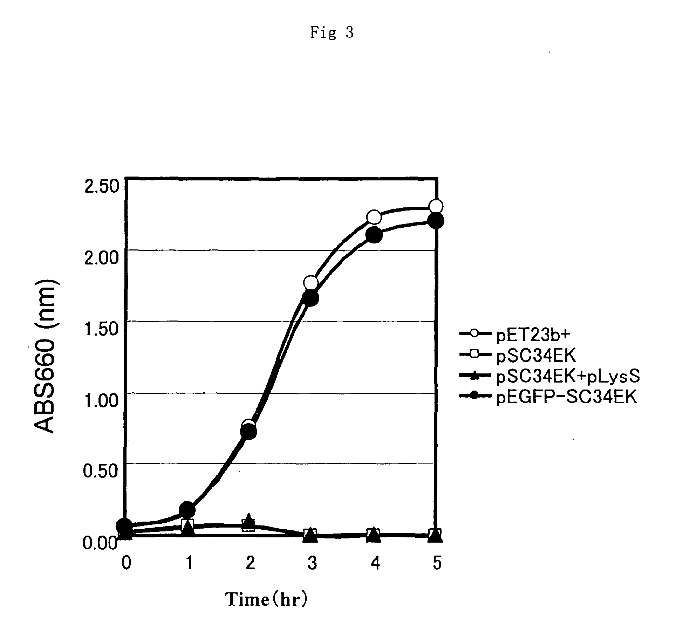

The host *E. coli* BL21 (DE3) strain was transformed with the pET23b+ and pSC34EK plasmids (Fig. 1) and colonies were formed on agar medium. While transformation with the pET23b+ plasmid formed colonies about 1 to 3 mm, the colonies formed by transformation with the pSC34EK plasmid did not grow more than 1 mm, indicating some kind of growth inhibition by the introduction of the pSC34EK plasmid. The pSC34EK plasmid-incorporated *E. coli* colonies were precultured by inoculating the cells in LB liquid medium and the cells were subjected to a main culture. Then, the number of cells was measured at 1-hour intervals. After two hours, absorbance was only about 0.05 to 0.2, and after addition of IPTG, the cell growth stopped and the cells agglutinated, making it impossible to collect cells (Fig. 3). By contrast, the strain with the pET23b+ plasmid had an absorbance (ABS660) of 0.4 to 0.8 after two hours of the main culture, accompanied by steady cell growth after addition of IPTG, and absorbance exceeded 2.0 after five hours (Fig. 3). This suggests that the sole expression of SC34EK peptide is toxic and inhibits *E. coli* growth. It became clear from this that the simple incorporation of SC34EK in the high expression system of *E. coli* is not sufficient to produce SC34EK in *E. coli.*

For more strict expression control, the host *E. coli* was transformed with the pLysS plasmid and pSC34EK plasmid. The *E. coli* with the plasmids were subjected to a preculture and a main culture as above. After two hours of the main culture, the absorbance rose to 0.1 to 0.2, a slight improvement from the foregoing system; however, the addition of IPTG stopped propagation and the cells agglutinated (Fig. 3). The fact that the pLysS plasmid in the host did not produce notable effects revealed that the SC34EK peptide is more toxic than initially thought.

This called for a method of SC34EK peptide production non-harmful to the *E. coli* cell. The inventors of the present invention thought to express a fusion protein including the SC34EK peptide flanked by an EGFP protein (Biochem Biophys Res Commun. 1996 Oct 23; 227(3):707-11) and Histag, and constructed a pEGFP-SC34EK plasmid (Fig. 1) for expressing such a protein. When transformed with this plasmid, the cells formed colonies about 1 to 3 mm as in the case of the pET23b+ plasmid, and there was no growth inhibition by the introduction of the pEGFP-SC34EK-Histag plasmid. As above, the strain with the pEGFP-SC34EK plasmid was subjected to a preculture and a main culture. After two hours of the main culture, absorbance (ABS660) was 0.4 to 0.8, accompanied by steady growth after addition of IPTG, and the absorbance exceeded 2.0 after 5 hours, showing almost the same growth pattern as the strain with the pET23b+ plasmid (Fig. 3). This experiment found that the expression of the SC34EK peptide as a fusion protein with a EGFP protein can neutralize toxicity, making it possible to produce SC34EK at a high yield without arresting *E. coli* growth.

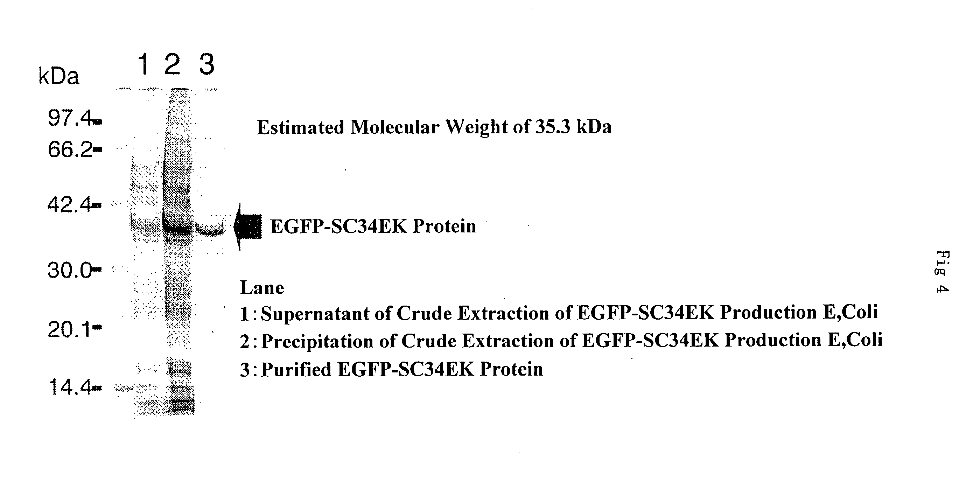

The strain with the pEGFP-SC34EK-Histag plasmid was incubated, and proteins were produced by subjecting the *E. coli* BL21 (DE3) strain to a main culture. The *E. coli* cells were collected by centrifugation and the cells were disrupted to extract proteins. The solution of extracted proteins was further centrifuged to obtain a supernatant sample and a precipitate. The supernatant sample was purified with a nickel column to obtain a purified sample. The purified sample fluoresced green under UV light (wavelength: 220 to 340 nm), showing the presence of EGFP protein. Further, the fact that purification was possible with the nickel column indicated that the protein includes the Histag region. By SDS-PAGE analysis of the purified sample, a band with a molecular weight of (35.3 kDa) was detected, as expected for the chimeric protein including the SC34EK peptide between the EGFP protein and Histag (Fig. 4). Because the EGFP protein and Histag protein were produced in *E. coli* without degradation, it was shown that protein production from the SC34EK peptide domain flanked by these two proteins was also possible without degradation.

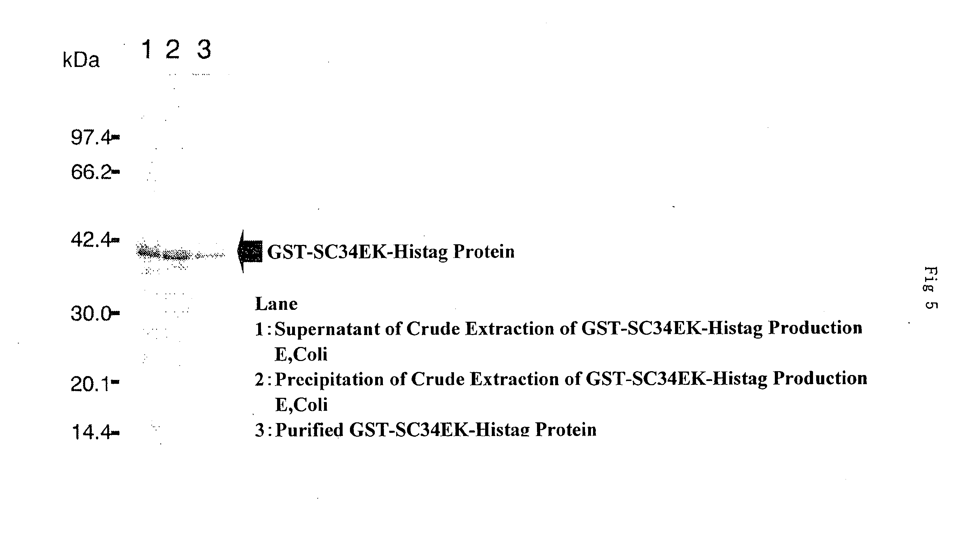

To determine whether the same result would be obtained by fusion with other proteins, an assessment was made as to the expression of a fusion protein including SC34EK peptide flanked by a GST (glutathione-S-transferase) protein and Histag. Since the gene encoding GST protein was already present in the pET41b+ plasmid, the pGST-SC34EK-Histag plasmid was constructed by adding the coding gene fragment of the SC34EK peptide (Fig. 2). Using the same procedure used for the pEGFP-SC34EK-Histag plasmid, the cells were transformed with the pGST-SC34EK-Histag plasmid, and proteins were produced by subjecting the *E. coli* BL21(DE3) strain to a main culture. After incubation, the *E. coli* cells were collected and disrupted, and then purified with a nickel column. The size of colonies, patterns of absorbance change, and the yield of GST-SC34EK-Histag protein were similar to the results using the pEGFP-SC34EK plasmid. Further, Histag purification was possible as above, and the purified protein matched the expected molecular weight in SDS-PAGE analysis. The fact that the experiment using the pGST-SC34EK-Histag plasmid yielded essentially the same result as that using the pEGFP-SC34EK plasmid suggests that the toxicity of the SC34EK peptide can also be neutralized when the SC34EK peptide is expressed as a fusion protein with GST protein. Further, since purification of the fusion protein was possible with a nickel column and the purified sample had specific binding to the glutathione column using GST protein as a tag (GST-tag), and from the matched molecular weight in SDS-PAGE analysis, it was shown that non-degradable protein production from the SC34EK peptide domain was also possible by fusion expression with the GST protein and Histag protein (Fig. 5).

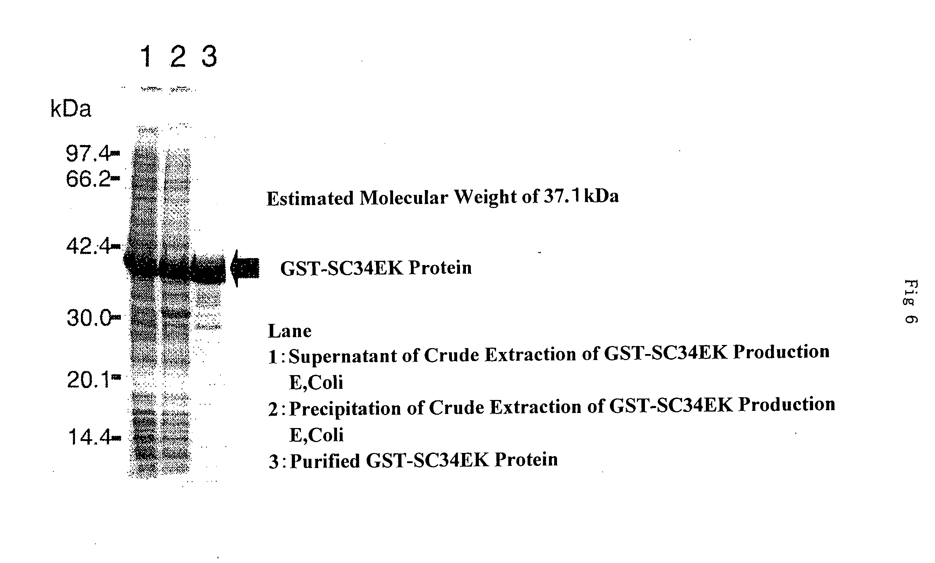

Finally, an assessment was made as to expression of a fusion protein including the SC34EK peptide and GST protein, excluding the Histag region. Specifically, the terminal cysteine residue of the gene fragment encoding the SC34EK peptide was modified to a stop codon (Gene Fragment II digested with restriction enzymes EcoRI and HindIII), and this fragment was incorporated in pET41b+ to construct the expression plasmid pGST-SC34EK (Fig. 2) encoding the protein. The plasmid was deposited with the International Patent Organism Depositary, National Institute of Advanced Industrial Science and Technology (Central 6, 1-1-1 Higashi, Tsukuba, Ibaraki, Japan; Accession No. FERM P-20910, deposited May 12, 2006; International Accession No. FERM BP-10868, July 13, 2007). The cells were transformed and purified with a glutathione column after incubation. SDS-PAGE analysis of the purified sample showed a band with the expected molecular weight (37.1 kDa), confirming successful purification of the GST-SC34EK fusion protein with the glutathione column, and non-degradation of the SC34EK peptide domain (Fig. 6).

With this system using *E. coli*, the cost of producing one tonne of SC34EK peptide can be reduced to 1 billion yen - 1/100,000 of the cost, 10 trillion yen, required for the production of the same amount by chemical synthesis.

### Example 2: Production of SC34EK Peptide Using Yeast Hosts Host

- Sake Yeast
- K7-U: (a/α, ura3/ura3)
   Strain K7 (Kyokai No. 7) yeast was subjected to EMS mutagenesis. Colonies that grew in 5'-FOA medium (2% glucose, 0.67% yeast nitrogen base) were selected as an ura3 mutant strain and used as a host.
- K7-T: (a/α, trp1/trp1)
   Strain K7 (Kyokai No. 7) yeast, tryptophan auxotroph, trp1 mutant strain
- Laboratory Yeast
   YNN27 (α, trp1, ura3, gall) was used as a host.

### Construction of SC34EK Peptide Recombinant Vector

### Secretion vector

- YEp-FLAG1 Vector (Sigma)
- pRS424-ADH1 (TRP1 marker)
- pRS426-ADH1 (URA3 marker)

### Chromosome-Integrated Vector

- pRS406 (URA3 marker)

### Primers (1) Used for Preparation of SC34EK Peptide (×5); YEp-FLAG Vector

### SC34EK-5S

SEQ ID NO: 10
(GAGAAGAACGAGAAGGAGCTCAAGTGCTGGATGGAGTGGGACCGCAAGGATCGAGGAGTACACC
AAGAAGATCGAGGAGCTCATCAAGAAGTCCCAGGAGCAGCAG)

### SC34EK-5A

SEQ ID NO: 11
(GATCTTGCGGTCCCACTCCTACCAGCACTTGAGCTCCTTCTCGTTCTTCTCCTGCTGCTCCTGG
GACTTCTTGATGAGCTCCTCGATCTTCTTGGTGTACTCCTC)

PCR Conditions
· 96°C (5 min) 1 cycle
· 96°C (20 sec), 60°C (30 sec), 72°C (5 min), 20 cycles
· 72°C (7 min), 1 cycle

PCR amplification was performed using LA-Taq (Takara Bio Inc.).

As a result, DNA fragments of a target size were obtained.

The PCR amplification products were separated by agarose gel electrophoresis, and extracted with a QIAquick Gel Extraction kit (Qiagen).

Using the purified PCR fragment as a template, PCR was performed as follows.

### Linker Addition

N5-F (N-terminus)
SEQ ID NO: 12 (GGAATTCGTCGACTTACTC)
C5-R (C-terminus)
SEQ ID NO: 13 (GGAATTCGTCGACTTACTC)
PCR Conditions
· 96°C (5 min), 1 cycle
· 96°C (20 sec), 50°C (30 sec), 72°C (5 min), 20 cycles
· 72°C (7 min), 1 cycle

The obtained molecule was cut with XhoI and SalI, and purified with a QIAquick PCR Purification kit (Qiagen).

After purification, the PCR fragment was cut with the XhoI and SalI of YEpFLAG, and ligated to transform JM109. From colonies that grew on LBA plates, a vector with the target fragment inserted was obtained.

### Primers (2) Used for Preparation of SC34EK Peptide (×1); YEp-FLAG vector

### SC34EK-1YF

SEQ ID NO: 14
(TGTTGGATGGAATGGGATAGGAAGATTGAAGAATACACTAAGAAGATTGAAGAATTGATTAAGA
AGTCTCAAGAA)

### SC34EK-1YR

SEQ ID NO: 15
(ACACTTCAATTCCTTTTCGTTCTTTTCTTGTTGTTCTTGAGACTTCTTAATCAATTCTTCAATC
TTCTTAGTGTA)

Using the primers SC34EK-1YF and SC34EK-1YR, PCR was performed with KOD-Plus-DNA Polymerase (Toyobo).

### PCR Conditions

· 94°C (2 min), 1 cycle
· 94°C (15 sec), 68°C (30 sec), 30 cycles

The resulting PCR products were purified with a QIAquick PCR Purification kit (Qiagen), and used as template for the next round of PCR, for which KOD-Plus-DNA Polymerase was used.
SC34EK-F2 (EcoRI)
SEQ ID NO: 16 (CCCGGAATTCTGTTGGATGGAATGGGATAG)
SC34EK-R2 (SalI)
SEQ ID NO: 17 (ACGCGTCGACACACTTCAATTCCTTTTCGTTC)
PCR Conditions
· 94°C (2 min), 1 cycle
· 94°C (15 sec), 50°C (30 sec), 68°C (30 sec), 30 cycles
· 68°C (30 sec), 1 cycle

The resulting PCR fragments of a desirable size were cut with the restriction enzymes EcoRI and SalI, and then purified with a QIAquick PCR Purification kit (Qiagen). The fragment was then ligated to the vector obtained by cutting the YEp-FLAG vector with EcoRI and SalI, using a ligation kit Ver. 2 (Takara Bio Inc.). The vector was inserted into LM109 competent cells for transformation. From the resulting colonies, a plasmid with the target DNA fragment inserted was purified.

### Yeast Transformation

The plasmids were transformed into the K7-T strain and the YNN27 strain, using an ordinary method.

The transformants were plated on a plate agar containing minimal medium + amino acid mix (-Trp) supplemented with 2%agar (2% glucose, 0.67% YNB; yeast nitrogen base), and colonies that grew in the amino acid mix (40 mg/l adenine, 20 mg/l L-arginine, 100 mg/ml 1-aspartic acid, 100 mg/ glutamic acid, 20 mg/l L-histidine, 60 mg/l L-leucine, 30 mg/l L-lysine, 20 mg/l l-methionine, 50 mg/l L-phenylalanine, 375 mg/l L-serine, 200 mg/l threonine, 30 mg/l L-tyrosine, 150 mg/l L-valine, 20 mg/l uracil) were incubated as single colonies on the same medium.

### Induction of SC34EK Peptide Production

The cells were shake cultured in a liquid medium (minimal medium + amino acid mix (-Trp)) at 30°C for 1 to 2 days, followed by another shake culturing for 2 days in induction medium (3% glycerol, 2% EtOH, 0.67% YNB + amino acid (-trp).

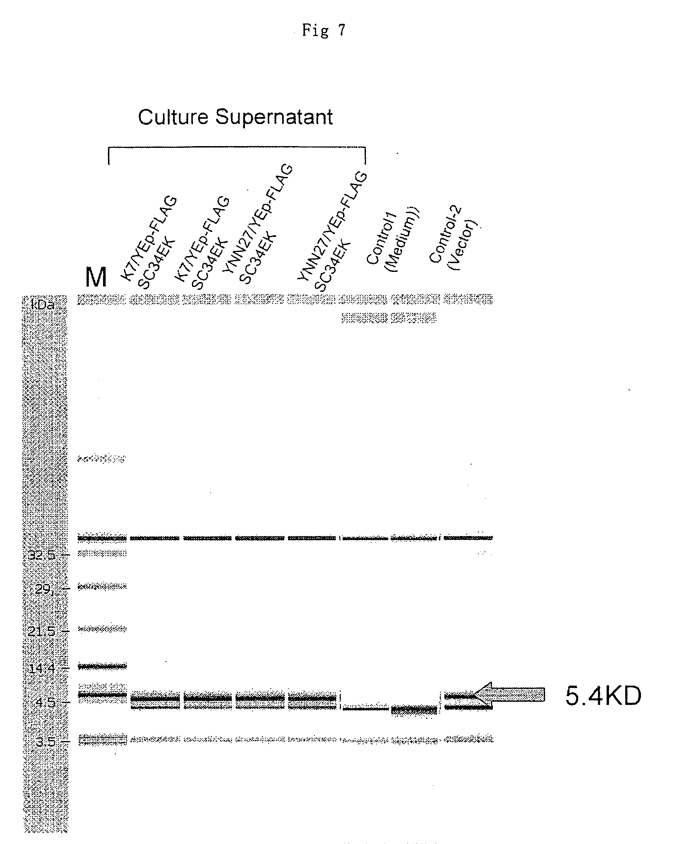

The cultures were subjected to centrifugal filtration (MW3000; MILLIPORE), concentrated 50 times, and subjected to electrophoresis and western blotting. The results of electrophoresis are shown in Fig. 7.

### Secretion vector: pRS424-ADH1 (TRP1 Marker), pRS426-ADH1 (URA3 Marker) Alpha-Factor Signal Sequence, and SC34EK Peptide

PAF1Fw: SEQ ID NO: 18 (ATTAAAAGAATGAGATTTCCTTCAATTTTT)
PAF2Rv: SEQ ID NO: 19 (GCTGGCAATAGTAGTATTTATAAACAATAA)

As a template, the genomic DNA of K7 was used.
· 96°C (5 min), 1 cycle
· 96°C (20 sec), 50°C (30 sec), 72°C (5 min), 20 cycles
· 72°C (7 min), 1 cycle

PCR amplification was performed using LA-Taq (Takara Bio Inc.)

As a result, DNA fragments of a target size were obtained. The PCR amplification products were separated by agarose gel electrophoresis, and extracted with a QIAquick Gel Extraction kit (Qiagen).

Using the purified PCR fragment as a template, PCR was performed as follows.
PAF2Fw: SEQ ID NO: 20 (CGGGATCCATTAAAAGAATGAGATTTCCTT)
PAF2Rv: SEQ ID NO: 21 (CGGAATTCGCTGGCAATAGTAGTATTTATA)
· 96°C (5 min), 1 cycle
· 96°C (20 sec), 50°C (30 sec), 72°C (5 min), 20 cycles
· 72°C (7 min), 1 cycle

PCR amplification was performed using LA-Taq (Takara Bio Inc.)

As a result, DNA fragments of a target size were obtained. The PCR fragments were cut with BamHI and EcoRI, and used for ligation after purification with a QIAquick PCR Purification kit (Qiagen).

### Amplification of SC34EK (×1) Peptide

The purified fragment of the PCR amplified products obtained with the primers (2) used for the preparation of SC34EK peptide (x1) was used as a template to perform PCR.
PAF3Fw: SEQ ID NO: 22 (ACGCGTCGACTGTTGGATGGAATGGGATAG)
PAF3Rv: SEQ ID NO: 23 (TGCGGTCGACACACTTCAATTCCTTTTCGTT)
· 96°C (5 min), 1 cycle
· 96°C (20 sec), 50°C (30 sec), 72°C (5 min), 20 cycles
· 72°C (7 min), 1 cycle

PCR amplification was performed using LA-Taq (Takara Bio Inc.)

The resulting target PCR fragment was cut with SalI, and purified with a QIAquick PCR Purification kit (Qiagen) for ligation.

The fragment after purification was treated with the restriction enzymes BamHI and EcoRI of pRS416-ADH1, and the fragment (BamHI-EcoRI) obtained by PCR with PAF2Fw and PAF2Rv was ligated using a Ligation Kit. Ver. 2 (Takara Bio Inc.). By transforming *E. coli* JM109, a target plasmid (pRS416-ADH-AF) was obtained. The plasmid was purified, and the fragment obtained by digestion with the restriction enzyme SalI and using the primers Paf3Fw and PAF3Rv (SalI restriction enzyme treatment) was ligated. By transforming *E. coli* JM109, a target plasmid (pRS416-GPDAFS1) was obtained.

The plasmid was transformed into the K7Δura strain and YNN27 strain.

### Amplification of SC34EK (×5) Peptide

PAF4Fw: SEQ ID NO: 24 (ACGCGTCGACGAGAAGAACGAGAAGGAGCT)
PAF4Rv: SEQ ID NO: 25 (CGGAATTCGCGATCTTGCGGTCCCACTCCTA)

PCR was performed under the following conditions. As a template, the PCR fragment amplified with the primers (1) used for the preparation of the SC34EK peptide (×5) was used.
· 96°C (5 min), 1 cycle
· 96°C (20 sec), 55°C (30 sec), 72°C (5 min), 20 cycles
· 72°C (7 min), 1 cycle

PCR amplification was performed using LA-Taq (Takara Bio Inc.).

The resulting fragment was treated with the restriction enzyme SalI, and purified with a QIAquick PCR Purification kit (Qiagen). After purification, the fragment was ligated to pRS416-ADH-AF treated with the restriction enzyme SalI. By transforming *E. coli* JM109, a target plasmid was obtained. After purification, the plasmid was transformed into yeasts YNN27 and K7Δura.

### Integrated Vector: pRS406 (URA3 marker)

The α factor signal sequence of secretion vector pRS424-SC34EK and the SC34EK (×1) BamHI-SalI fragment were excised and inserted into the BamHI-SalI site of pRS406. By transforming the JM109 strain, a target plasmid was obtained from colonies that grew in LBA. After purification, the plasmid was used to transform yeast.

### Yeast Transformation

The plasmid was transformed into the K7-U strain and YNN27 strain, by an ordinary method.

The transformants were plated on a plate of agar containing minimal medium + amino acid mix (Δura) supplemented with 2% agar (2% glucose, 0.67% YNB; yeast nitrogen base), and colonies that grew in the amino acid mix -Ura (40 mg/l adenine, 20 mg/l L-arginine, 100 mg/ml 1-aspartic acid, 100 mg/ glutamic acid, 20 mg/l L-histidine, 60 mg/l L-leucine, 30 mg/l L-lysine, 20 mg/l 1-methionine, 50 mg/l L-phenylalanine, 375 mg/l L-serine, 200 mg/l threonine, 30 mg/l L-tyrosine, 150 mg/l L-valine, 200 mg/l tryptophan) were incubated as single colonies on the same medium.

### Induction of SC34EK Peptide Production

The cells were shake cultured in liquid medium (minimal medium + amino acid mix (-Ura)) at 30°C for 1 to 2 days, followed by another shake culturing for 2 days in induction medium (3% glycerol, 2% EtOH, 0.67% YNB + amino acid (-Urp)).

The cultures were centrifuged with a filter (MW3000; MILLIPORE) and concentrated 50 times.

### Western Blotting

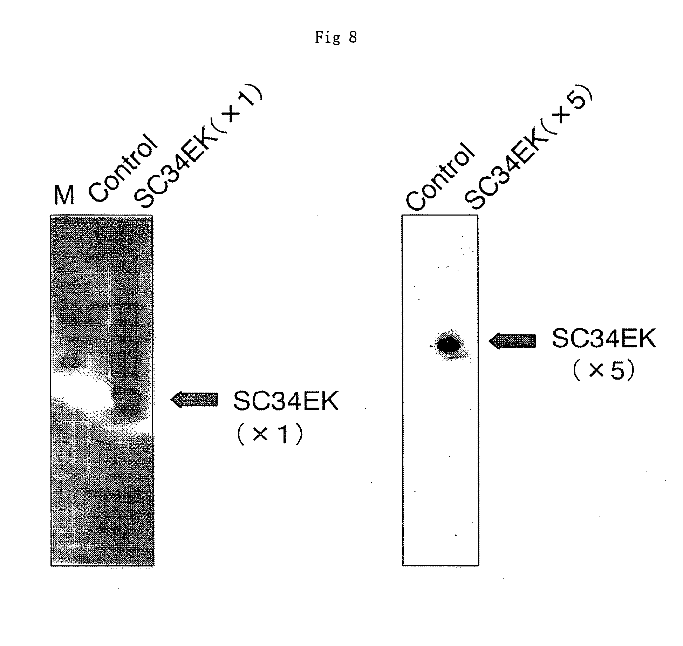

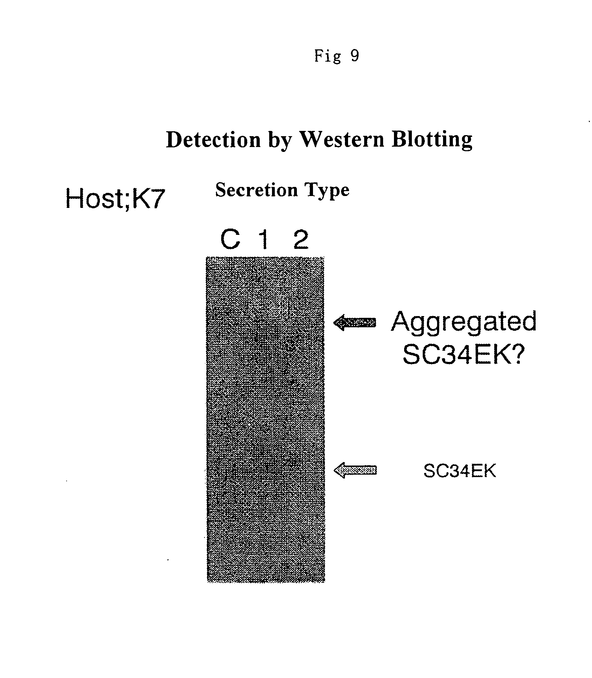

SC34EK (×1) peptide and SC34EK (×5) peptide were detected using an anti-SC34EK antibody. The samples were electrophoresed on a gel (16% Wide-PAGE mini Peptide-PAGE mini; tricine-based; Tefco), using an electrophoresis buffer (upper buffer: 1,000 ml containing 12.1 g tris, 17.9 g tricine, and 1.0 g SDS; lower buffer: 1,000 ml containing 24.2 g tris-HCl, pH 8.9), under a constant voltage of 125 mV for 1.5 hours. The peptides on the gel were transferred to Hybond-P (Amersham) using a semidry blotter (Amersham), which took 1 hour under 65 mA. After the transfer, peptides were detected with an ECL-plus western blotting detection system (Amersham), using an anti-SC34EK antibody and an anti-rabbit antibody. The SC34EK (×5) peptide was detected as a single specific band, and detection of SC34EK (×1) peptide was also possible (Fig. 8).
The chromosome-integrated sample formed a band larger than the target size (Fig. 9).

### Preparation of Antibody

Synthetic Peptide Used as Antigen for Preparation of Antibody SEQ ID NO: 26:
Ac-Cys(SH)-Gly-Gly-Gly-Trp-Met-Glu-Trp-Asp-Arg-Lys-Ile-Glu-Glu-Tyr-Thr-Lys-Lys-Ile-Glu-Glu-Leu-Ile-Lys-Lys-Ser-Gln-Glu-Gln-Gln-Glu-Lys-Asn-Glu-Lys-Glu-Leu-Lys-NH2
Molecular weight: 4670.2 Da

Using the synthetic peptide, a custom antibody was prepared by a commercial supplier (Invitrogen). By immunizing a rabbit, an anti-SC34EK antibody was prepared. The anti-SC34EK antibody was purified by affinity chromatography using the synthetic peptide.

### Example 3: SC34EK Production Using Aspergillus Host

### Expression of Glucoamylase-Fused SC34EK in Aspergillus sp. Aspergillus Host

As the *Aspergillus* (*Aspergillus oryzae*) host used for genetic transformation, a leucine-auxotrophic mutant *Aspergillus* strain, *Aspergillus oryzae* leu-5 (deposited with the International Patent Organism Depositary, National Institute of Advanced Industrial Science and Technology; Accession no. FERM P-20079, deposited June 7, 2004), obtained from *Aspergillus oryzae* O-1013 (deposited with the International Patent Organism Depositary, National Institute of Advanced Industrial Science and Technology; Accession no. FERM P-16528, deposited November 20, 1997) by known UV radiation mutagenesis was used.

### Selectable Marker Plasmid

As a selectable marker, *Aspergillus* nidulans-derived gene ANleu2 (SEQ ID NO: 27) that encodes β-isopropylmalate dehydrogenase, capable of complementing the leucine auxotrophy mutation in the leucine-auxotrophic mutant *Aspergillus* strain *Aspergillus oryzae* leu-5, was used. ANleu2 is a gene with two introns, and it encodes a β-isopropylmalate dehydrogenase of 370 amino acid residues. The bases 1 to 139, 320 to 1549, and 1550 to 3560 of SEQ ID NO: 27 are a promoter region, an open reading region, and a terminator region, respectively. The two introns reside in bases 795 to 851 and 1273 to 1332 in the open reading region in bases 320 to 1549 of SEQ ID NO: 27. The amino acid sequence is represented by SEQ ID NO: 28. ANleu2 was amplified by PCR using LA-Taq (Takara Bio Inc.), using genomic DNA of *Aspergillus nidulans* as a template. As the primers for ANleu2, primer P1: SEQ ID NO: 29 (5'-TGCCAGTTTTACCAGCTTGACC-3') and primer P2: SEQ ID NO: 30 (5'-CTTTCATGTCATGTCCCTAGAAG-3') were used.

### PCR Conditions

· 96°C (5 min), 1 cycle
· 96°C (20 sec), 60°C (30 sec), 72°C (5 min), 30 cycles
· 72°C (7 min), 1 cycle

PCR was used to successfully amplify suitable genomic gene products. The PCR amplification products were subjected to phenol-chloroform extraction, followed by ethanol precipitation. After treatment, the amplification products were separated by agarose gel electrophoresis and extracted with a QIAquick Gel Extraction kit (Qiagen). The resulting gene fragments metA, hisA, ANhisA, ANleu2, and ANleu2B had adenine base overhang at 3' end of the products amplified by LA-Taq. The fragments were treated at 4°C for 20 hours to ligate to the T vector pGEM-T (Promega), using T4 DNA ligase (Promega). The ligation solution was then transformed into *E. coli* JM109 competent cells (Takara Bio Inc.). Transformants were obtained by isolating white colonies, using LB medium supplemented with ampicillin, IPTG, and X-gal.

A plasmid was prepared from each transformant by an ordinary method. ANleu2-subcloned plasmids were denominated as pANLA.

### Transformation of Aspergillus

Transformation of the *Aspergillus* leucine-auxotrophic mutant strain *Aspergillus oryzae* leu-5 with pANLA was performed by an ordinary, protoplast-PEG-calcium method (Mol. Gen. Genet., 218, 99-104, (1989)). Protoplasts were prepared by collecting *Aspergillus oryzae* leu-5 with a glass filter 3G1 after incubation in GPY (2% glucose, 1% polypeptone, 0.5% yeast extract) at 30°C for 1 day, and causing the cells to react at 30°C for 3 hours in a protoplast forming solution containing 0.8 M NaCl (also containing 5 mg/ml Yatalase (Takara Bio Inc.), 5 mg/ml cellulase (Wako Pure Chemical Industries, Ltd.), and 5 mg/ml lysing enzyme (Sigma)). The filtrate passed through a 3G2 glass filter was used as a protoplast solution in the ordinary protoplast-PEG-calcium method. In the protoplast-PEG-calcium method, plasmids can be added by ordinary cotransformation, in which a selectable marker-containing plasmid, such as pANLA, and any other plasmid containing no selectable marker are added in any proportion to insert the plasmid fragments into the chromosome of the transformants selected in minimal medium. As the selective medium for the transformants, Czapek-Dox minimal medium (glucose 3%, magnesium sulfate 0.1%, dipotassium hydrogenphosphate 0.1%, potassium chloride 0.05%, sodium nitrate 0.3%, ferrous sulphate 0.00001 M, 0.8 M NaCl, 1.5% agar, pH 6.3) was used. Transformants were obtained after 7 days of incubation at 30°C.

### 1) Expression in Liquid Culture

### Construction of Expression Plasmid

For the production of SC34EK in *Aspergillus sp*. in liquid culture, a start codon ATG was joined downstream of the sodM promoter of *Aspergillus sp*., and the coding gene of SC34EK was ligated immediately following ATG. However, this failed to produce SC34EK either inside or outside of the cell, revealing that SC34EK production in *Aspergillus sp*. is unachievable by simply using the high expression system of *Aspergillus sp*. alone.

Attempts were made to cause expression of a fusion gene containing glucoamylase gene glaB (cDNA) and an SC34EK equivalent gene, under the control of the sodM promoter, which is strongly expressed in liquid cultures of *Aspergillus sp.* The sequence of the sodM promoter is represented by SEQ ID NO: 31, the cDNA sequence of the glucoamylase gene glaB by SEQ ID NO: 32, the sequence of the SC34EK coding gene by SEQ ID NO: 33, and the sequence of the glaB terminator by SEQ ID NO: 34. The amino acid sequence inferred from the cDNA sequence of glaB (SEQ ID NO: 32) is represented by SEQ ID NO: 35, and the amino acid sequence synthesized based on the coding gene of SC34EK (SEQ ID NO: 33) is represented by SEQ ID NO: 36. The Cys at amino acid 1 and cys at amino acid 36 in SEQ ID NO: 36 are amino acid residues provided for excision by chemical cleavage, and the sequence from amino acids 2 to 35 of SEQ ID NO: 36 is the main functional part required for anti-HIV binding inhibition activity.

The SC34EK can be fused with the glaB for glucoamylase by, for example, joining the start codon and subsequent sequence of glaB cDNA downstream of the sodM promoter, and substituting a part of the base sequence of glaB cDNA, corresponding to a specific amino acid sequence, with the coding gene of SC34EK, and finally inserting a glaB terminator downstream of the stop codon of glaB cDNA. For example, the sequence from amino acid 331 (glycine) to amino acid 365 (glutamine) in SEQ ID NO: 35 of glaB glucoamylase may be substituted with the sequence of SEQ ID NO: 36. However, the site of substitution is not limited to this, and the gene may be incorporated by insertion, instead of substitution.

The following describes how the sequence from amino acid 331 (glycine) to amino acid 365 (glutamine) in SEQ ID NO: 35 of glaB glucoamylase is substituted with the sequence of SEQ ID NO: 36. In the first round of PCR, gene products were amplified, as follows.
sodM promoter: *Aspergillus oryzae* O-1013 genomic DNA obtained by an ordinary method was used as a template. Primers: P3 (5'-TTATGTACTCCGTACTCGGTTGAATTATTA-3'; SEQ ID NO: 37) and P4 (5'-TGTTCCGCATTTTGGGTGGTTTGGTTGGTA-3'; SEQ ID NO: 38).
glaB cDNA (SEQ ID NO: 32), bases 1 to 991: A glaB cDNA-subcloned plasmid was used as a template. Primers: P5 (5'-ACCACCCAAAATGCGGAACAACCTTCTTTT-3'; SEQ ID NO: 39) and P6 (5'-CCATCCAGCACCACTGCCGCGGCCTTTCCT-3'; SEQ ID NO: 40).
SC34EK (SEQ ID NO: 33), full length: A plasmid subcloning SEQ ID NO: 33 was used as a template. Primers: P7 (5'-GCGGCAGTGGTGCTGGATGGAGTGGGACCG-3'; SEQ ID NO: 41) and P8 (5'-TTCACTTGACGCACTTGAGCTCCTTCTCGT-3'; SEQ ID NO: 42).
glaB cDNA (SEQ ID NO: 32), bases 1097 to 1482: A glaB cDNA-subcloned plasmid was used as a template. Primers: P9 (5'-GCTCAAGTGCGTCAAGTGAACGTCAGTGAA-3'; SEQ ID NO: 43) and P10 (5'-GAAAGTACATCTACCACGACCCAACAGTTG-3'; SEQ ID NO: 44).
glaB terminator, full length (SEQ ID NO: 34): Primers: P11 (5'-GTCGTGGTAGATGTACTTTCCAGTGCGTGT-3'; SEQ ID NO: 45) and P12 (5'-GCGAACAGAGCTATACCTTCACATACCTTC-3'; SEQ ID NO: 46).
PCR amplification was performed using LA-Taq (Takara Bio Inc.).

### PCR Conditions

· 96°C (5 min), 1 cycle
· 96°C (20 sec), 60°C (30 sec), 72°C (5 min), 30 cycles
· 72°C (7 min), 1 cycle

As a result, four suitable genomic gene products were obtained. The four PCR amplification products were separated by agarose gel electrophoresis and extracted with a QIAquick Gel Extraction kit (Qiagen). After extraction, the four samples were subjected to ethanol precipitation together. Then, using a mixture of the four samples as a template, the second round of PCR was performed with primers P3 and P12. PCR amplification was performed using LA-Taq (Takara Bio Inc.).

### PCR Conditions

· 96°C (5 min), 1 cycle
· 96°C (20 sec), 68°C (5 min), 30 cycles
· 72°C (7 min), 1 cycle

As a result, a single suitable genomic gene product was obtained. The PCR amplification product was separated by agarose gel electrophoresis and extracted with a QIAquick Gel Extraction kit (Qiagen). The PCR product had adenine sticking out from the ends, and was ligated to a T vector, pGEM-T (Promega), by treating it at 4°C for 20 hours using T4 DNA ligase (Promega). The ligation solution was transformed into *E. coli* JM109 competent cells (Takara Bio Inc.). Transformants were obtained by isolating white colonies, using LB medium supplemented with ampicillin, IPTG, and X-gal. As a result, a plasmid pMGSC1 was obtained that had genes for expressing the fusion gene in which the sequence from amino acid 331 (glycine) to amino acid 365 (glutamine) of glaB glucoamylase in SEQ ID NO: 35 was substituted with the sequence of SEQ ID NO: 36.

As a control, a plasmid was constructed that was necessary to produce the full-length glucoamylase in liquid medium. The plasmid was constructed by performing amplification as follows.
sodM promoter: *Aspergillus oryzae* O-1013 genomic DNA obtained by an ordinary method was used as a template. Primers: P3 (5'-TTATGTACTCCGTACTCGGTTGAATTATTA-3'; SEQ ID NO: 37) and P4 (5'-TGTTCCGCATTTTGGGTGGTTTGGTTGGTA-3'; SEQ ID NO: 38).
glaB cDNA (SEQ ID NO: 32), full length: A glaB cDNA-subcloned plasmid was used as a template. Primers: P5 (5'-ACCACCCAAAATGCGGAACAACCTTCTTTT-3'; SEQ ID NO: 39) and P10 (5'-GAAAGTACATCTACCACGACCCAACAGTTG-3'; SEQ ID NO: 44).
glaB terminator, full length (SEQ ID NO: 34): *Aspergillus oryzae* O-1013 genomic DNA was used as a template: Primers: P11 (5'-GTCGTGGTAGATGTACTTTCCAGTGCGTGT-3'; SEQ ID NO: 45) and P12 (5'-GCGAACAGAGCTATACCTTCACATACCTTC-3'; SEQ ID NO: 46).
PCR amplification was performed using LA-Taq (Takara Bio Inc.).

### PCR Conditions

· 96°C (5 min), 1 cycle
· 96°C (20 sec), 60°C (30 sec), 72°C (5 min), 30 cycles
· 72°C (7 min), 1 cycle

As a result, three suitable genomic gene products were obtained. The four PCR amplification products were separated by agarose gel electrophoresis and extracted with a QIAquick Gel Extraction kit (Qiagen). After extraction, the three samples were subjected to ethanol precipitation together. Then, using a mixture of the three samples as a template, the second round of PCR was performed with primers P3 and P12. PCR amplification was performed using LA-Taq (Takara Bio Inc.).

### PCR Conditions

· 96°C (5 min), 1 cycle
· 96°C (20 sec), 68°C (5 min), 30 cycles
· 72°C (7 min), 1 cycle

As a result, a single suitable genomic gene product was obtained. The PCR amplification product was separated by agarose gel electrophoresis and extracted with a QIAquick Gel Extraction kit (Qiagen). The PCR product had adenine sticking out from the ends, and was ligated to a T vector, pGEM-T (Promega), by treating it at 4°C for 20 hours using T4 DNA ligase (Promega). The ligation solution was transformed into *E. coli* JM109 competent cells (Takara Bio Inc.). Transformants were obtained by isolating white colonies, using LB medium supplemented with ampicillin, IPTG, and X-gal. As a result, a plasmid pMG1 was obtained that had genes for expressing the full-length glaB glucoamylase (SEQ ID NO: 35).

### Transformant Liquid Culture and Product Confirmation

An ordinary protoplast-PEG-calcium method was used to transform *Aspergillus sp*. leucine-auxotrophic mutant strain *Aspergillus oryzae* leu-5 with pANLA alone, or cotransform with pANLA and pMGSC1, or pANLA and pMG1. The presence or absence of the gene in the transformants was confirmed by PCR.

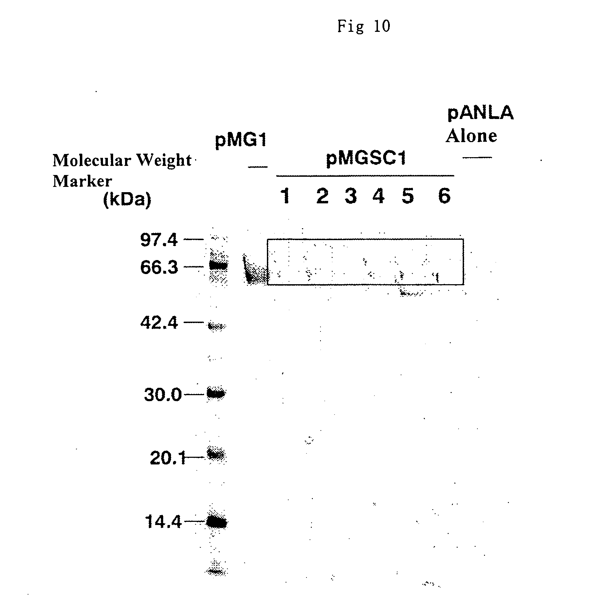

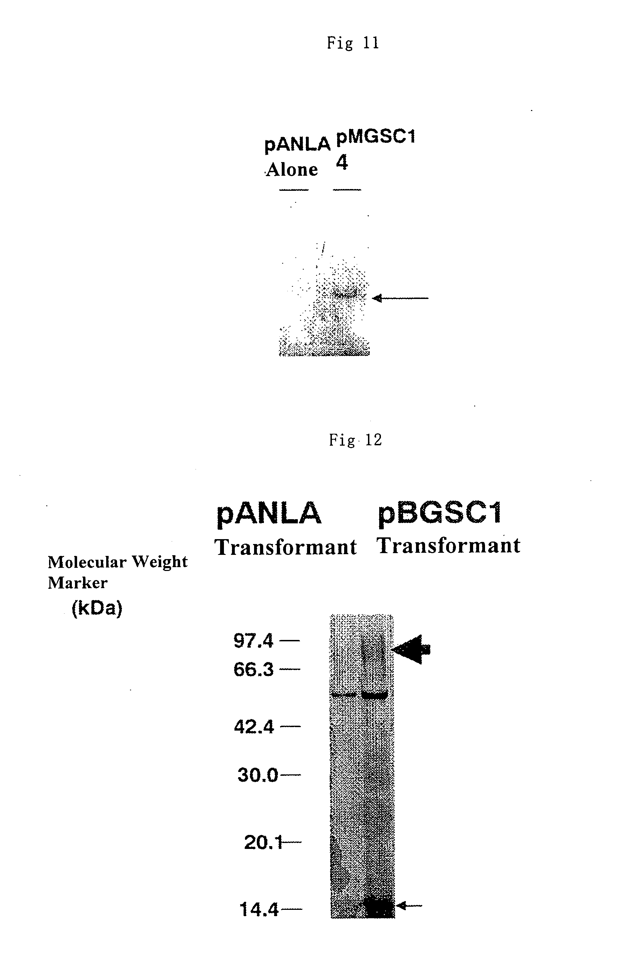

The presence or absence of the SC34EK coding gene in the transformants was confirmed by PCR. As a result, a strain transformed with pMGSC1, a strain transformed with pMG1 (control), and a strain transformed only with pANLA (control) were obtained. The transformants were incubated in GPY liquid medium (40 ml) at 30°C for 3 days, and cells were collected with a glass filter and incubated in 100 ml of Czapek-Dox liquid medium (glucose 3%, magnesium sulfate 0.1%, dipotassium hydrogenphosphate 0.1%, potassium chloride 0.05%, sodium nitrate 0.3%, ferrous sulphate 0.00001 M, pH 6.3) for 1 day. After 1 day of incubation, 10 µl of the culture was analyzed by SDS-PAGE and coomassie staining, which found secretory expression of a protein believed to be the fusion protein of glucoamylase and SC34EK, as indicated by the solid lines in Fig. 10. To further confirm this, western analysis was performed for the pANLA transformant culture and the pMGSC1 transformant culture 4 (corresponding to lane 4 in Fig. 10), as shown in Fig. 11. As a result, a signal was found only in the pMGSC1 transformant, indicating extracellular secretion of SC34EK as a fusion protein. Further, the result of ELISA quantification using the anti-SC34EK antibody showed that about 100 mg of the secreted protein was the SC34EK peptide per 1 L of the medium. It was therefore found that mass production of SC34EK by secretion is possible through the cultivation of *Aspergillus sp*. in liquid medium.

### 2) Expression in Solid Culture

### Construction of Expression Plasmid

Attempts were made to cause expression of a fusion gene containing the glucoamylase gene glaB (cDNA) and an SC34EK equivalent gene, under the control of the glaB promoter, which is strongly expressed in solid cultures of *Aspergillus sp.* The sequence of the glaB promoter is represented by SEQ ID NO: 47, the cDNA sequence of the glucoamylase gene glaB by SEQ ID NO: 32, the sequence of the SC34EK coding gene by SEQ ID NO: 33, and the sequence of the glaB terminator by SEQ ID NO: 34. The amino acid sequence inferred from the cDNA sequence of glaB (SEQ ID NO: 32) is represented by SEQ ID NO: 35, and the amino acid sequence synthesized based on the coding gene of SC34EK (SEQ ID NO: 33) is represented by SEQ ID NO: 36. The Cys at amino acid 1 and cys at amino acid 36 in SEQ ID NO: 36 are amino acid residues provided for excision by chemical cleavage, and the sequence (amino acids 2 to 35) of SEQ ID NO: 36 is the main functional part required for anti-HIV binding inhibition activity.

SC34EK can be fused with the glaB for glucoamylase by, for example, joining the start codon and subsequent sequence of glaB cDNA downstream of the sodM promoter, and substituting a part of the base sequence of the glaB cDNA, corresponding to a specific amino acid sequence, with the coding gene of SC34EK, and finally inserting a glaB terminator downstream of the stop codon of the glaB cDNA. For example, the sequence from amino acid 331 (glycine) to amino acid 365 (glutamine) in SEQ ID NO: 35 of glaB glucoamylase may be substituted with the sequence of SEQ ID NO: 36. However, the site of substitution is not limited to this, and the gene may be incorporated by insertion, instead of substitution.

The following describes how the sequence from amino acid 331 (glycine) to amino acid 365 (glutamine) in SEQ ID NO: 35 of glaB glucoamylase is substituted with the sequence of SEQ ID NO: 36. In the first round of PCR, gene products were amplified as follows.
glaB promoter: *Aspergillus oryzae* O-1013 genomic DNA obtained by an ordinary method was used as a template. Primers: P13 (5'-TCTCAACCCAAGTAACGATGAAGAATGGCT-3'; SEQ ID NO: 48) and P14 (5'-TGTTCCGCATGATGGTGGTGACTTCCAAGA-3'; SEQ ID NO: 49).
glaB cDNA (SEQ ID NO: 32), bases 1 to 991: A glaB cDNA-subcloned plasmid was used as a template: Primers: P15 (5'-CACCACCATCATGCGGAACAACCTTCTTTT-3'; SEQ ID NO: 50) and P6 (5'-CCATCCAGCACCACTGCCGCGGCCTTTCCT-3'; SEQ ID NO: 40).
SC34EK (SEQ ID NO: 33), full length: A plasmid subcloning SEQ ID NO: 33 was used as a template. Primers: P7 (5'-GCGGCAGTGGTGCTGGATGGAGTGGGACCG-3'; SEQ ID NO: 41) and P8 (5'-TTCACTTGACGCACTTGAGCTCCTTCTCGT-3'; SEQ ID NO: 42).
glaB cDNA (SEQ ID NO: 32), bases 1097 to 1482: A glaB cDNA-subcloned plasmid was used as a template. Primers: P9 (5'-GCTCAAGTGCGTCAAGTGAACGTCAGTGAA-3'; SEQ ID NO: 43) and P10 (5'-GAAAGTACATCTACCACGACCCAACAGTTG-3'; SEQ ID NO: 44).
glaB terminator, full length (SEQ ID NO: 34): *Aspergillus oryzae* O-1013 genomic DNA was used as a template. Primers: P11 (5'-GTCGTGGTAGATGTACTTTCCAGTGCGTGT-3'; SEQ ID NO: 45) and P12 (5'-GCGAACAGAGCTATACCTTCACATACCTTC-3'; SEQ ID NO: 46).
PCR amplification was performed using LA-Taq (Takara Bio Inc.).

### PCR Conditions

· 96°C (5 min), 1 cycle
· 96°C (20 sec), 60°C (30 sec), 72°C (5 min), 30 cycles
· 72°C (7 min), 1 cycle

As a result, four suitable genomic gene products were obtained. The four PCR amplification products were separated by agarose gel electrophoresis and extracted with a QIAquick Gel Extraction kit (Qiagen). After extraction, the four samples were subjected to ethanol precipitation together. Then, using a mixture of the four samples as a template, a second round of PCR was performed with primers P13 and P12. PCR amplification was performed using LA-Taq (Takara Bio Inc.).

### PCR Conditions

· 96°C (5 min), 1 cycle
· 96°C (20 sec), 68°C (5 min), 30 cycles
· 72°C (7 min), 1 cycle

As a result, a single suitable genomic gene product was obtained. The PCR amplification product was separated by agarose gel electrophoresis and extracted with a QIAquick Gel Extraction kit (Qiagen). The PCR product had adenine sticking out from the ends, and was ligated to a T vector, pGEM-T (Promega), by treating it at 4°C for 20 hours using T4 DNA ligase (Promega). The ligation solution was transformed into *E. coli* JM109 competent cells (Takara Bio Inc.). Transformants were obtained by isolating white colonies, using LB medium supplemented with ampicillin, IPTG, and X-gal. As a result, a plasmid pBGSC1 was obtained that had genes for expressing the fusion gene in which the sequence from amino acid 331 (glycine) to amino acid 365 (glutamine) of glaB glucoamylase in SEQ ID NO: 35 was replaced with the sequence of SEQ ID NO: 36.

### Transformant Solid Culture and Product Confirmation

An ordinary protoplast-PEG-calcium method was used to transform *Aspergillus sp*. leucine-auxotrophic mutant strain *Aspergillus oryzae* leu-5 with pANLA alone, or cotransform with pANLA and pBGSC1. The presence or absence of the gene in the transformants was confirmed by PCR.

The presence or absence of the SC34EK coding gene in the transformants was confirmed by PCR. As a result, a strain transformed with pBGSC1 and a strain transformed only with pANLA (control) were obtained.

The transformants were cultured on malted rice solid medium, for which steamed rice with 70% milled rice was used. The medium was inoculated with the spores of transformants (spores on a GPY plate suspended in starch), and the cells were incubated at 30°C for 2 days, and 37°C for 1 day. After incubation, the cultures were extracted with a 0.5% NaCl solution (5 times the amount of culture) for 3 hours at room temperature, and 10 µl of the extract was analyzed by SDS-PAGE and coomassie staining, which found secretory expression of a protein believed to be the fusion protein of glucoamylase and SC34EK, as indicated by the arrow in Fig. 12. To further confirm this, ELISA was performed using the SC34EK peptide expressed in *E. coli* as a standard. As a result, it was found that about 330 mg of the secreted protein was SC34EK peptide, per 1 kg of the malted rice. It was therefore found that mass production of SC34EK by secretion is possible through the cultivation of *Aspergillus sp*. on a solid medium.

### Purification of Fusion Protein Produced by Solid Culture

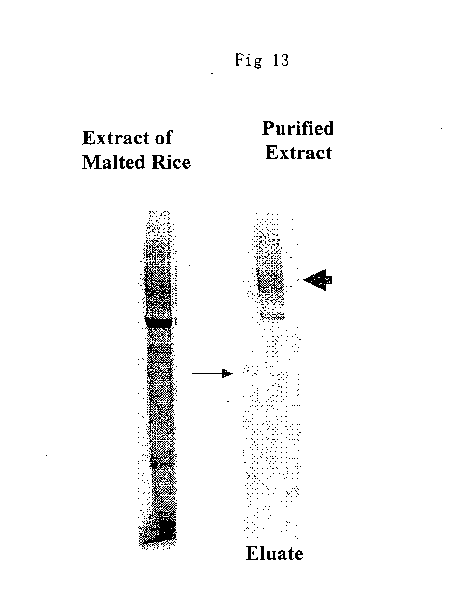

0.5 kg of the malted rice culture of the pBGSC1 transformant was extracted with 2,500 ml of 0.5% NaCl solution for 3 hours at room temperature. The extract was centrifuged at 10,000 rpm and the supernatant was collected. Then, ammonium sulfate was added to the supernatant (90% saturated), and the mixture was allowed to stand at room temperature for 20 hours. The supernatant was collected after centrifugation at 10,000 rpm. Then, butyl toyopearl resin (Amersham Pharmacia) was added to the supernatant in a batch. The mixture was allowed to stand at room temperature for 30 minutes and the resin was collected. After washing with 2 M ammonium sulfate, the resin was suspended in an equivalent amount of water, and the supernatant was collected. The simple ammonium sulfate treatment and the batch addition of hydrophobic resin increased the purity of the fusion protein by 80% or more compared with the purity of the malted rice extract, as shown by the results of SDS-PAGE in Fig. 13. This is highly advantageous industrially in terms of cost.

With this system using *Aspergillus sp*., the cost of producing one tonne of SC34EK peptide can be reduced to 2 billion yen - 1/5,000 of the cost, 10 trillion yen, required for the production of the same amount by chemical synthesis.

### Example 4: Excision of N36-Binding Peptide from Fusion Protein

The fusion proteins obtained according to the methods of Examples 1, 2, and 3 were agitated at 37°C for 2 hours with a solution containing 0.1 M acetic acid, 10mM DTT, and 6 M urea (or 6 M guanidine hydrochloride). Then 1-cyano-4-dimethylaminopyridinium salt (DMAP-CN) was added to the mixture in an amount 5 times the equivalent of thiol. By allowing for a reaction at room temperature for 15 minutes, S-cyanylated proteins were obtained. After the reaction, the reaction mixture was dialyzed overnight with 50% acetic acid, and the resulting solution was lyophilized. The lyophilized powder was dissolved again in a 6 M urea (or 6 M guanidine hydrochloride) solution, and 25% ammonium water was added to make the final concentration 3 M. The mixture was allowed to react at 15°C for 15 minutes. Excision may be done using 0.05 M sodium hydroxide. After the reaction, the pH was adjusted to 6.0 with acetic acid. The reaction mixture was passed through a Unison UK-C18 (150 mm × 10 mm, Imtakt) equilibrated with 0.05% formic acid. After adsorption and washing, the mixture was eluted with a step gradient of 20 to 50% B (B: acetonitrile) to collect a peptide fraction with an amidated C terminal (carboxyl group when sodium hydroxide is used). The fraction was lyophilized to obtain a lyophilized powder of the peptide.

### Example 5: Quantification and Activity Assay of Products Containing the SC34EK Sequence

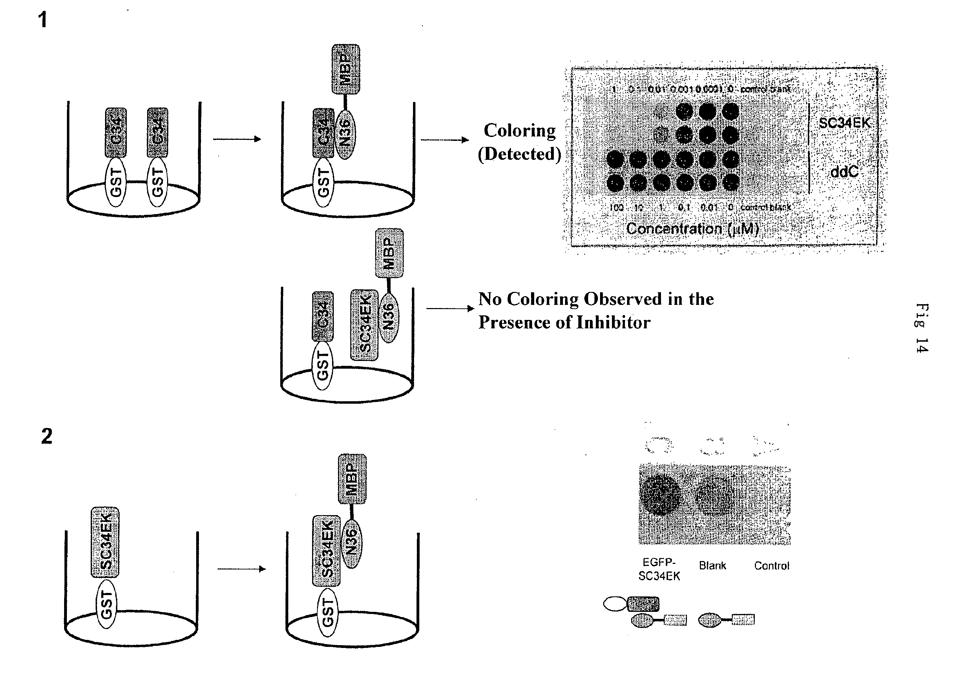

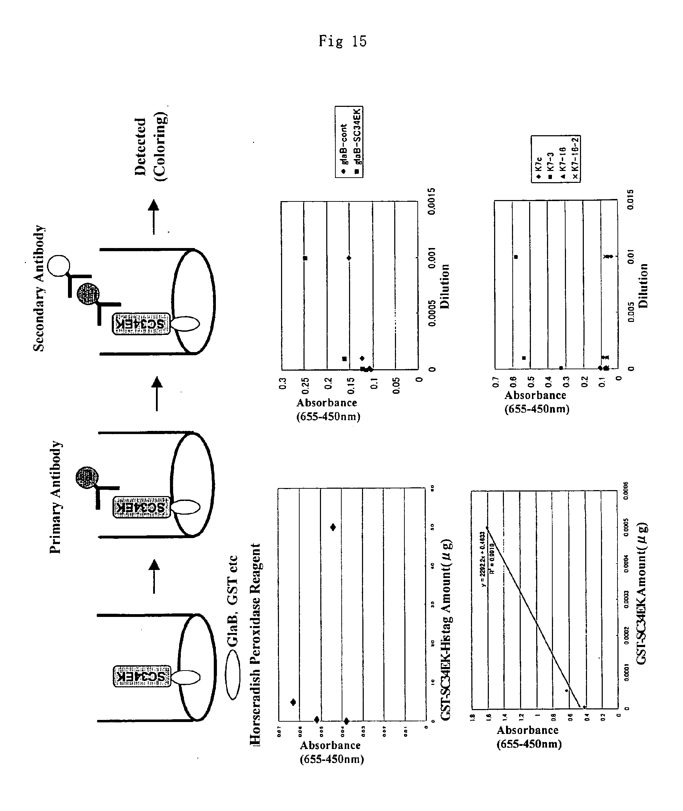

### ELISA Quantification (Figs. 14 and 15)

Quantification of the SC34EK-sequence-containing fusion protein and SC34EK was made using an anti-SC34EK antibody.

The fusion protein was diluted with Na₂CO₃ (0.060 M Na₂CO₃, 0.136 M NaHCO₃, pH 9.6), and dispensed into an ELISA plate (Sumiron), 50 ml in each well. The samples were allowed to stand at 4°C overnight or at room temperature for 2 hours, and washed three times with 200 µl of wash Buffer (50 mM Tris-HCl, 0.2 % Tween 20, pH 8.0). Then, 200 µl of blocking buffer (1 % BSA/wash buffer) was dispensed into each well, where the samples were allowed to stand at 4°C for 2 to 3 hours, and washed three times with 200 µl of washing buffer. The primary antibody (anti-SC34EK antibody) was diluted 5,000 times with wash buffer, and 50 µl of the diluted antibody was added to the samples, which were then allowed to stand at room temperature for 2 hours. After washing the samples three times with 200 µl of wash buffer, 50 µl of the secondary antibody (anti-rabbit IgG (H+L)) and 50 µl of biotin antibody (Funakoshi Corporation) were added after 1:5,000 dilution with wash buffer. The mixture was allowed to stand at room temperature for 2 hours, and washed six times with 200 µl of wash buffer. A TMP peroxidase substrate kit (Bio-Rad) was used to elicit a signal, and the absorbance at 655 nm was measured. Then, the reaction was stopped with 100 µl of 1 M sulfuric acid, and the absorbance at 450 nm was measured. The absorbance 450 nm - 655 nm was determined, and a standard curve was created using the GST-SC34EK produced in Example 1 as a standard, so as to quantify the products containing the SC34EK sequence produced in the microbe.

### N36-Binding Activity (ELISA Method)

The binding reaction between SC34EK and N36 was measured to confirm the activity of the SC34EK fusion protein.
EGFP-SC34EK produced in Example 1 was diluted with Na₂CO₃ buffer (pH 9.6), and 50 µl of the diluted samples was dispensed onto an ELISA well plate, where the samples were allowed to stand at room temperature for 2 hours or at 4°C overnight. The samples were washed three times with 200 µl of wash buffer (PBS buffer containing 0.025 volume% Tween 20). Then, 200 µl of blocking buffer (wash buffer containing 0.1 weight% BSA) was dispensed into each well, and the mixture was allowed to stand at 4°C for 3 hours, and then washed with 200 µl of wash buffer three times. MBP-N36 (Maltose Binding Protein-N36) was diluted 1,000 times with wash buffer, and 100 µl of the diluted protein was dispensed into each well, where the mixture was allowed to stand at 37°C for 1 hour and 30 minutes. After washing the protein six times with 200 µl of wash buffer, 100 µl of anti-MBP antibody was dispensed into each well after 1:1,000 dilution with wash buffer. The mixture was allowed to stand at 4°C for 1 hour. After adding 100 µl of Blue Phos (KPL) to each well, the samples were allowed to stand at 37°C for 20 minutes to measure absorbance at 650 nm. As a result, EGFP-SC34EK was shown to have N36 binding activity.

### N36-C34-Binding Inhibition Reaction

### Inhibitor Activity Assay Using a N36-C34-Binding Reaction

GST-C34 was diluted with Na₂CO₃ buffer (pH 9.6), and 50 µl was dispensed into an ELISA well plate, where it was allowed to stand at room temperature for 2 hours or at 4°C overnight to adsorb. This was followed by washing three times with 200 µl of wash buffer (PBS containing 0.025 volume% Tween 20). Then, 200 µl of blocking buffer (wash buffer containing 0.1 weight% BSA) was dispensed into each well, and the mixture was allowed to stand at 4°C for 2 to 3 hours, and then washed with 200 µl of wash buffer three times. MBP-N36 was diluted 1,000 times with washing buffer, and the N36-binding inhibitor produced in Example 1 was diluted in series by a factor of 10. Then, 100 µl of the sample dilutions was dispensed into each well, where the samples were allowed to stand at 37°C for 1 hour and 30 minutes, and washed six times with 200 µl of wash buffer. Thereafter, 100 µl of antibody (monoclonal anti-maltose binding protein) was dispensed into each well after 1:1,000 dilution with wash buffer. The samples were then allowed to stand at 4°C for 1 hour. After adding 100 µl of the Blue Phos colorizer (KPL) to each well, the mixture was allowed to stand at 37°C for 20 minutes to measure absorbance at 650 nm. By the suppressed signal, the EGFP-SC34EK was shown to serve as an inhibitor and have N36-C34-binding inhibition activity (Fig. 14).

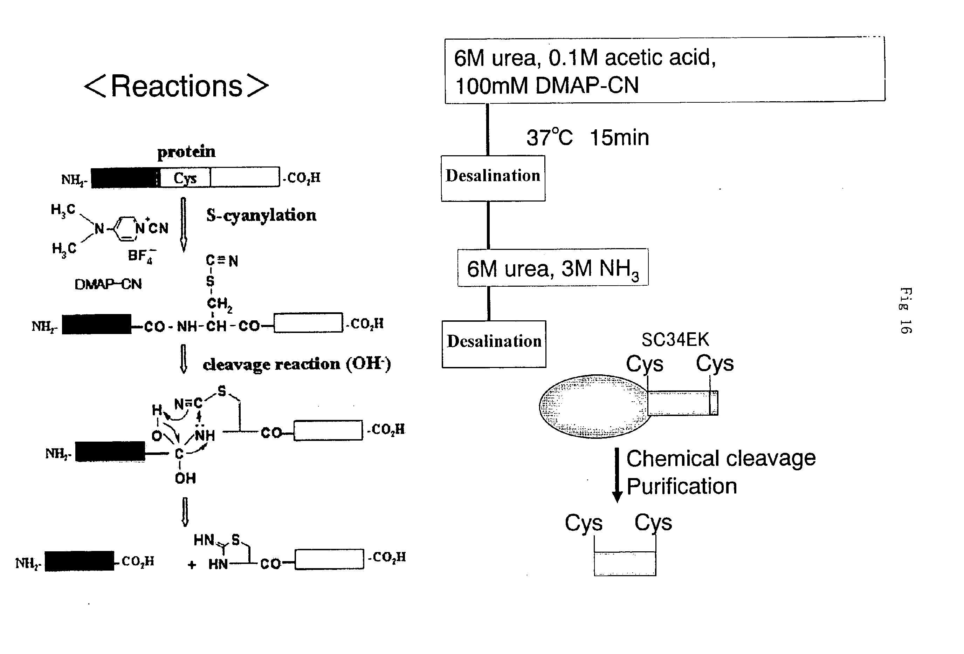

Fig. 16 shows a schematic illustration of the Cys cleavage reaction.

### Anti-HIV Activity Assay by MAGI (Multinuclear Activation of Galactosidase Indicator) Assay

Using MAGI cells produced by expressing CD4-LTR/βgal in Hela cells, the anti-HIV activities of samples containing the SC34EK sequence produced in microbe were measured by MAGI assay.
MAGI cells were treated with trypsin for 5 minutes, and an equivalent or greater amount of DMEM (Dulbecco's Modified Eagle's Medium) was added. By counting, the number of cells was adjusted to 1 × 10⁴ cells/well over 96 wells. HIV-1 strain clone NL4-3 was diluted with DMEM, and 100 µl was added to each well. Then, products containing the SC34EK sequence produced in microbe, and the GST-SC34EK solution described in Example 1 were diluted in series by a factor of 10, and added to each well. After 48 hours of incubation, X-Gal was added and the number of cells that were stained blue was counted. Since the cells are stained blue by X-GAL when infected by HIV, the concentration at 50% staining was denoted as EC50 (Effective concentration).

**[Table 1]**

| | |
|---|---|
| GST-SC34EK | EC50 |
| Sample | (ng/ml) |
| MAGI assay | 1.10 ±0.5²⁵ |

### [Industrial Applicability]

The present invention is applicable to fields relating to the production of SC34EK peptide.

## Claims

1. A method for producing an N36-binding peptide, the method comprising:
introducing a recombinant vector incorporating a DNA molecule encoding the N36-binding peptide that binds to an N36 protein derived from a retrovirus that causes immunodeficiency in a mammal into *E. coli* as a host to produce a transformant,
culturing the transformant in a medium to produce and accumulate the N36-binding peptide in the culture, and
collecting the N36-binding peptide from the culture.

2. The method according to claim 1, wherein said transformant is cultured in said medium, and the N36-binding peptide is produced and accumulated in the culture by intracellular expression, periplasm expression or secretion expression.

3. The method according to claim 2, wherein said transformant is cultured in said medium and the N36-binding peptide is produced and accumulated by intracellular expression of *E. coli.*

4. The method for producing an N36-binding peptide according to claim 1, wherein said recombinant vector contains the DNA molecule encoding an N36-binding peptide fused with a DNA molecule encoding a protein that can be expressed in *E. coli.*

5. The method for producing an N36-binding peptide according to claim 1, wherein said recombinant vector contains the DNA molecule encoding an N36-binding peptide fused with a DNA molecule encoding a protein that can be expressed in *E. coli,*
said DNA molecule encoding an N36-binding peptide containing at one end or both ends thereof a DNA molecule encoding a site capable of being recognized to be cleaved at a peptide bond.

6. The method for producing an N36-binding peptide according to claim 5, wherein said recognition site is cysteine.

7. The method for producing an N36-binding peptide according to claim 4, wherein said protein that can be expressed in *E. coli* is at least one member selected from the group consisting of glutathione S transferase and EGFP.

8. A method for producing an N36-binding peptide, the method comprising:
introducing a recombinant vector incorporating a DNA molecule encoding an N36-binding peptide that binds to an N36 protein derived from a retrovirus that causes immunodeficiency in a mammal into a filamentous fungus as a host to produce a transformant,
culturing the transformant in a medium to produce and accumulate the N36-binding peptide in the culture, and
collecting the N36-binding peptide from the culture.

9. The method for producing an N36-binding peptide according to claim 8, wherein said filamentous fungus is *Aspergillus oryzae*.

10. The method for producing an N36-binding peptide according to claim 8, wherein said recombinant vector contains the DNA molecule encoding the N36-binding peptide fused with a DNA molecule encoding a protein that can be expressed in the filamentous fungus.

11. The method for producing an N36-binding peptide according to claim 8, wherein said recombinant vector contains the DNA molecule encoding an N36-binding peptide fused with a DNA molecule encoding a protein that can be expressed in the filamentous fungus,
said DNA molecule encoding the N36-binding peptide containing at one end or both ends thereof a DNA molecule encoding a site capable of being recognized to be cleaved at a peptide bond.

12. The method for producing an N36-binding peptide according to claim 11, wherein said recognition site is cysteine.

13. The method for producing an N36-binding peptide according to claim 10, wherein said protein that can be expressed in the filamentous fungus is glucoamylase.

14. The method for producing an N36-binding peptide according to claim 8, wherein the filamentous fungus is cultured in a solid medium.

15. A method for producing an N36-binding peptide, the method comprising:
introducing a recombinant vector incorporating a DNA molecule encoding an N36-binding peptide that binds to an N36 protein derived from a retrovirus that causes immunodeficiency in a mammal into a yeast as a host to produce a transformant, culturing the transformant in a medium to produce and accumulate the N36-binding peptide in the culture, and
collecting the N36-binding peptide from the culture.

16. The method for producing an N36-binding peptide according to claim 15, wherein said yeast is a microorganism of the genus Saccharomyces.

17. The method for producing an N36-binding peptide according to claim 15, wherein said vector contains the DNA molecule encoding an N36-binding peptide and, fused thereto, a DNA molecule encoding a protein that can be expressed in the yeast.

18. The method for producing an N36-binding peptide according to claim 15, wherein said recombinant vector contains the DNA molecule encoding an N36-binding peptide and, fused thereto, a DNA molecule encoding a protein that can be expressed in the yeast,
said DNA molecule encoding an N36-binding peptide containing at one end or both ends thereof a DNA molecule encoding a site capable of being recognized to be cleaved at a peptide bond.

19. The method for producing an N36-binding peptide according to claim 18, wherein said recognition site is cysteine.

20. The method for producing an N36-binding peptide according to claim 17, wherein said protein that can be expressed in the yeast is alpha-factor.

21. A preventive or therapeutic agent composition for a retrovirus infection that causes immunodeficiency in a mammal, the composition containing as an effective component the N36-binding peptide obtained by the method of any one of claims 1 to 20.

22. The preventive or therapeutic composition according to claim 21, wherein the retrovirus that causes immunodeficiency in a mammal is human immunodeficiency virus (HIV).


### (Sequence listing)

SEQUENCE LISTING

<110> Gekkeikan Sake Co., Ltd.

<120> Method for producing N36-binding peptide

<130> P07-87

<140> PCT/JP2007/064710
<141> 2007-07-26

<150> JP 2006-204827
<151> 2006-7-27

<160> 50

<170> PatentIn version 3.1

<210> 1
<211> 34
<212> PRT
<213> Artificial Sequence

<400> 1

Trp Met Glu Trp Asp Arg Lys Ile Glu Glu Tyr Thr Lys Lys Ile Glu
1               5                   10                  15

Glu Leu Ile Lys Lys Ser Gln Glu Gln Gln Glu Lys Asn Glu Lys Glu
            20                  25                  30

Leu Lys

<210> 2
<211> 108
<212> DNA

<213>  Artificial Sequence

<400>  2
tgctggatgg aatgggatcg caaaatcgaa gaatacacca aaaaaatcga agaactgatc      60

aaaaaaagcc aggaacagca ggaaaaaaac gaaaaagaac tgaaatgc                  108

<210>  3
<211>  70
<212>  DNA
<213>  Artificial Sequence

<400>  3
tgctggatgg aatgggatcg caaaatcgaa gaatacacca aaaaaatcga agaactgatc      60

aaaaaaagcc                                                             70

<210>  4
<211>  60
<212>  DNA
<213>  Artificial Sequence

<400>  4
gcatttcagt tctttttcgt ttttttcctg ctgttcctgg ctttttttga tcagttcttc      60

<210>  5
<211>  41
<212>  DNA
<213>  Artificial Sequence

<400>  5
cccggaattc gagcctcgag tgctggatgg aatgggatcg c                          41

<210>  6
<211>  29
<212>  DNA

<213>  Artificial Sequence

<400>  6
acgcgtcgac gcatttcagt tctttttcg                                    29

<210>  7
<211>  30
<212>  DNA
<213>  Artificial Sequence

<400>  7
ccgcggatcc gatggtgagc aagggcgagg                                   30

<210>  8
<211>  30
<212>  DNA
<213>  Artificial Sequence

<400>  8
cccggaattc ggcttgtaca gctcgtccat                                   30

<210>  9
<211>  33
<212>  DNA
<213>  Artificial Sequence

<400>  9
tttttaagct ttcatttcag ttctttttcg ttt                               33

<210>  10
<211>  106
<212>  DNA
<213>  Artificial Sequence

<400>  10
gagaagaacg agaaggagct caagtgctgg atggagtggg accgcaagga tcgaggagta    60

caccaagaag atcgaggagc tcatcaagaa gtcccaggag cagcag 106

<210> 11
<211> 105
<212> DNA
<213> Artificial Sequence

<400> 11
gatcttgcgg tcccactcct accagcactt gagctccttc tcgttcttct cctgctgctc 60

ctgggacttc ttgatgagct cctcgatctt cttggtgtac tcctc 105

<210> 12
<211> 19
<212> DNA
<213> Artificial Sequence

<400> 12
ggaattcgtc gacttactc 19

<210> 13
<211> 19
<212> DNA
<213> Artificial Sequence

<400> 13
ggaattcgtc gacttactc 19

<210> 14
<211> 75
<212> DNA
<213> Artificial Sequence

<400> 14
tgttggatgg aatgggatag gaagattgaa gaatacacta agaagattga agaattgatt 60

aagaagtctc aagaa 75

<210> 15
<211> 75
<212> DNA
<213> Artificial Sequence

<400> 15
acacttcaat tccttttcgt tcttttcttg ttgttcttga gacttcttaa tcaattcttc 60

aatcttctta gtgta 75

<210> 16
<211> 30
<212> DNA
<213> Artificial Sequence

<400> 16
cccggaattc tgttggatgg aatgggatag 30

<210> 17
<211> 32
<212> DNA
<213> Artificial Sequence

<400> 17
acgcgtcgac acacttcaat tccttttcgt tc 32

<210> 18
<211> 30
<212> DNA
<213> Artificial Sequence

<400> 18
attaaaagaa tgagatttcc ttcaattttt 30

<210> 19
<211> 30
<212> DNA
<213> Artificial Sequence

<400> 19
gctggcaata gtagtattta taaacaataa 30

<210> 20
<211> 30
<212> DNA
<213> Artificial Sequence

<400> 20
cgggatccat taaaagaatg agatttcctt 30

<210> 21
<211> 30
<212> DNA
<213> Artificial Sequence

<400> 21
cggaattcgc tggcaatagt agtatttata 30

<210> 22
<211> 30
<212> DNA
<213> Artificial Sequence

<400> 22
acgcgtcgac tgttggatgg aatgggatag 30

<210> 23
<211> 31

<212> DNA
<213> Artificial Sequence

<400> 23
tgcggtcgac acacttcaat tccttttcgt t 31

<210> 24
<211> 30
<212> DNA
<213> Artificial Sequence

<400> 24
acgcgtcgac gagaagaacg agaaggagct 30

<210> 25
<211> 31
<212> DNA
<213> Artificial Sequence

<400> 25
cggaattcgc gatcttgcgg tcccactcct a 31

<210> 26
<211> 38
<212> PRT
<213> Artificial Sequence

<400> 26

Cys Gly Gly Gly Trp Met Glu Trp Asp Arg Lys Ile Glu Glu Tyr Thr
1               5                   10                  15

Lys Lys Ile Glu Glu Leu Ile Lys Lys Ser Gln Glu Gln Gln Glu Lys
            20                  25                  30

Asn Glu Lys Glu Leu Lys
        35

<210> 27
<211> 3560
<212> DNA
<213> Aspergillus nidulans

<400> 27
tgccagtttt accagcttga ccagcacacg cttgcattac tttgccatga gccttccatc 60

aagactatat gccaccccgc tgttctccga agactcagat cagtatttat ccgtgacctg 120

agcctgagag cctatggagg tagagaacat agggaatacg accgagatag cgatatcggg 180

gataatccgt gggtgtactt ccccgcattt cggcctactc tccgactccg gcctgttgta 240

taaagatacg gattgtcgtt cgtctcaagt ctcaagtcac tttctgccag tacccctaca 300

cggtagctct agaaacatca tgtcagagaa gtcatacaac atcctcgtcc tccccggcga 360

cgggattggg cctgaagtta tggccgaagc caccaagatc ctctcccttt tcaacacatc 420

caccgtcaga ttcaggaccc agacggaact tatcggtggc tgtagcatcg acacccacgg 480

caaatcggtg acgcaggctg tcctcgatgc tgccgtatcc tctgatgcag tcctcttcgc 540

tgccgttgga ggcccaaagt gggaccatat ccgacgcgga ctcgacggac ccgagggagg 600

gctgctccag gtacggaagg ctatggacat ttatgcaaat ctgagaccgt gttccgttga 660

tagcccaagc agggagatcg ccagagactt cagccccttc aggcaggatg ttatagaggg 720

ggtcgacttc gtggtcgtga gggaaaattg tggaggcgca tactttggga agaaggttga 780

agaagacgat tatggttcgt tcttacctat acttacggtg acttgagtgg tttatatatt 840

gatgatacca gcaatggacg aatggggcta ctcagcctcc gaaatacagc ggattactcg 900

actctcggcg gagctcgcgc tgcgacacga tcctccctgg cctgtgatct ctctcgacaa 960

agccaacgtg ctggcctcgt cgcgtctctg gcgccgggtt gtcgaaaaga ccatgagcga 1020

ggaatacccg caggtgaagc ttgtccacca gcttgctgat tcggcgtcct taatcatggc 1080

cacgaacccg cgcgctctga acggcgtcat cttggcggac aatacctttg gcgatatggt 1140

atccgaccag gcaggctctc tggtcggaac gctgggtgtc ctgcctagcg cgagcttgga 1200

tggtttaccc aagccgggag agcagaggaa ggtacatggg ctctatgagc cgacgcatgg 1260

gtctgccccg acgtaggtca ttcccaaccc actactatcc cttcactcgt tgctcaccat 1320

aatgtatgat agaattgccg gtaagaacat cgccaacccg acagcgatga tcctctgcgt 1380

tgcgctgatg ttccggtact cgttcaacat ggaggccgag gcgcggcaga ttgaggctgc 1440

agtacgcact gttctcgata agggaattcg aacatctgat cttggaggtt cgacgggaac 1500

tagagagttt ggcgatgcgg ttgttgcggc gttgaaggga gagctctgat ctggtctagt 1560

tgggagcagt tagctaagct ggcgatggct gggcagtgac ttcaccgtat gaaatagagg 1620

atgatggcgt ccaatcattg aagcctcgtg gtaaggagct tggcatgagc ttgaaagctc 1680

tggagatctg cccatatatc cactgtcgcc caaccgggta ggtataatat ctgctgttgt 1740

gccagtcgct atagtcacca ctttatttaa gcagagctat cccacccaat atcggcattc 1800

tctgcaatat catgagtccc tgtaagacac taaatttaag atggtgatga tacatccgtt 1860

gttgatcgtc tcgtattcta tactccttac gcgatgacat caccgggatt acccaccatg 1920

acctcatctc agatcttgca aaacagttgc cctgagcaga atccctggga cgaattcatc 1980

atggttcctt ttttttgcca aatcacagtg caagccccct cttttctgca ctactacgtc 2040

gctatcagtt gtccatccgt catgctgagt ccaaacgctg gacgtcggtc ccctatttgt 2100

acttctatat gatgaccgtc tccatcgcct ttatcgcctc catcacctct cttatagatc 2160

tattcaacca atcttctcct catctctcct ttcactcgtc tccggctttt cagtacaatc 2220

caatattctt cgaccggcca agatgaccgt catctggggc ctcgatctcc atgagatttg 2280

tttcagcaag tttagatctg ccaacatgct caaccgggcg taccatctcc gtcgaacaaa 2340

gatggttgtc tatcagcttg caatgatcct ctgcgtctgc tcagaatccg tcgggacggc 2400

tgctttttca ggtacaggtt tctctccgca aacacctatc ctttccacaa tcgctttgcc 2460

tagacagtac tgacttcttt gggcgctttc gtgcagacta cctcgatcaa caaagctaca 2520

ttcaaggcca gcacccgggt gtgaaaatcc acaacaacag ctttattggc gcgttctcgt 2580

acaacgtctt tgtgggggtt gcagtggcgt ttatatttgg tgcagcgttc ttttttgacc 2640

tcttctggcc ggagaggcac gagtgcaaac atgtacgaat cagttggaag atttcggcag 2700

tagtcgttac tgttatgatg ctgagctcgg cgctgactat cacggtgagc cgttgtttaa 2760

ttattgatct accgcgcgcg atggtactga taatcaccgc aattcttagt gtatcggcgc 2820

tctacatgat gccgtcgtta caggaacaga tcgcgacact gcgcagacgt atagattgga 2880

gttctcgaag aaaccctctt acagtgcgtt atcatgcccg tgcccttcct gccatattct 2940

gacgcagacc agcataccga gataacccca aagtaattgc cgctgttgtt cttgcctggc 3000

ctgggtgggt attttgcgtt attaggtata tctattcctt taaattctcg ctgccatgcg 3060

ggtatgtgac tgatactttt ttttagtaca atcgtcctct tcatgtccca gaagcacgac 3120

gacaagtacg gtccaatgtc gaagtacggc cgtagtcagg aagacggcag tattgtggtc 3180

gataaggaag gacacaacag tcccgagcca acggcggcat aaataacacg ttacgaggtt 3240

acgatttact cctttctact cttttgtata caatatttcc gtttttggat ggctactgta 3300

ttgtactata gactgtagta ttgacggaac ttggcttcct gcaatgatac cctgttttgg 3360

gagaccggat ggcatttgcc atttgaactt gtatgcattc atacaggact ctactataat 3420

cgttcatgag ctcctggtag aaacggtgaa agatgtatac cttctagtaa agccataaac 3480

ccagggaagt aaattaagca ttgccaatgc cagcatatat catctatttg gcgcccactt 3540

ctagggacat gacatgaaag 3560

<210> 28
<211> 370
<212> PRT
<213> Aspergillus nidulans

<400> 28

Met Ser Glu Lys Ser Tyr Asn Ile Leu Val Leu Pro Gly Asp Gly Ile
1               5                   10                  15

Gly Pro Glu Val Met Ala Glu Ala Thr Lys Ile Leu Ser Leu Phe Asn
            20                  25                  30

Thr Ser Thr Val Arg Phe Arg Thr Gln Thr Glu Leu Ile Gly Gly Cys
        35                  40                  45

Ser Ile Asp Thr His Gly Lys Ser Val Thr Gln Ala Val Leu Asp Ala
    50                  55                  60

Ala Val Ser Ser Asp Ala Val Leu Phe Ala Ala Val Gly Gly Pro Lys
65                  70                  75                  80

Trp Asp His Ile Arg Arg Gly Leu Asp Gly Pro Glu Gly Gly Leu Leu
                85                  90                  95

Gln Val Arg Lys Ala Met Asp Ile Tyr Ala Asn Leu Arg Pro Cys Ser
            100                 105                 110

Val Asp Ser Pro Ser Arg Glu Ile Ala Arg Asp Phe Ser Pro Phe Arg
        115                 120                 125

Gln Asp Val Ile Glu Gly Val Asp Phe Val Val Val Arg Glu Asn Cys
    130                 135                 140

Gly Gly Ala Tyr Phe Gly Lys Lys Val Glu Glu Asp Asp Tyr Ala Met
145                 150                 155                 160

Asp Glu Trp Gly Tyr Ser Ala Ser Glu Ile Gln Arg Ile Thr Arg Leu
                165                 170                 175

Ser Ala Glu Leu Ala Leu Arg His Asp Pro Pro Trp Pro Val Ile Ser
            180                 185                 190

Leu Asp Lys Ala Asn Val Leu Ala Ser Ser Arg Leu Trp Arg Arg Val
        195                 200                 205

Val Glu Lys Thr Met Ser Glu Glu Tyr Pro Gln Val Lys Leu Val His
    210                 215                 220

Gln Leu Ala Asp Ser Ala Ser Leu Ile Met Ala Thr Asn Pro Arg Ala
225                 230                 235                 240

Leu Asn Gly Val Ile Leu Ala Asp Asn Thr Phe Gly Asp Met Val Ser
                245                 250                 255

Asp Gln Ala Gly Ser Leu Val Gly Thr Leu Gly Val Leu Pro Ser Ala
            260                 265                 270

Ser Leu Asp Gly Leu Pro Lys Pro Gly Glu Gln Arg Lys Val His Gly
        275                 280                 285

Leu Tyr Glu Pro Thr His Gly Ser Ala Pro Thr Ile Ala Gly Lys Asn
    290                 295                 300

Ile Ala Asn Pro Thr Ala Met Ile Leu Cys Val Ala Leu Met Phe Arg
305                 310                 315                 320

Tyr Ser Phe Asn Met Glu Ala Glu Ala Arg Gln Ile Glu Ala Ala Val
                325                 330                 335

Arg Thr Val Leu Asp Lys Gly Ile Arg Thr Ser Asp Leu Gly Gly Ser
            340                 345                 350

Thr Gly Thr Arg Glu Phe Gly Asp Ala Val Val Ala Ala Leu Lys Gly
        355                 360                 365

Glu Leu
    370

<210> 29
<211> 22
<212> DNA
<213> Artificial Sequence

<400> 29

tgccagtttt accagcttga cc                                                22

<210>  30
<211>  23
<212>  DNA
<213>  Artificial Sequence

<400>  30
ctttcatgtc atgtccctag aag                                               23

<210>  31
<211>  1038
<212>  DNA
<213>  sodM promoter

<400>  31
ttatgtactc cgtactcggt tgaattatta atcgggtatt gcattaactt aattagctat       60

gttcttcttt gttgaattag ccctaactct gggtagagtg tggtttttat agtgacatga      120

tggtggagga aggatttctt aattactagg gcagggttat tatgtggagt ttagctcatt      180

taggattctt gatggaaggt tcttatctcc ttgtcaatat ggtatcactc acatatattc      240

ttgaactata tcttcttgaa ttcctcggct atcctggtca gttcatgttc agtaccgtag      300

atagattgaa ttctggtcgt tcgtttctac tccttcatct tcagtccatt cagttcccta      360

ggttgttggt ctaccttcca tggccccggc tgttgtgggt tgcgtcatgt agatgaaggg      420

agctagccag atgataatat cagagttcta cagagtacca tgctaagcct ggggatttac      480

tggttaacat ttgcctagta acttgtaaga atcggaagta cttggctcca tgagccccac      540

attatgaaaa acaatggcat caacaaattg tctgtgcatt atcatagtat agttctgggt      600

gggggctata gtacttgctg gtacttcatg gagtgacagg ctgtagtagg cggccttaat      660

aaaacccagc agaacgcatt cattagacag atttatctac gcatatacca gctaacatag 720

cctatttgta atccttgtaa tccacactca cctacgaatc acatagacga aaggaagaat 780

gtgctcagtg gtgcgccaaa ggtcccccga atcccctcaa actcgctcat agtcacatcc 840

cgccaacatg attggtaaaa atgaaggtac cttgggggtc ctcagcatcc attccaggct 900

tatattctcc ctcgccataa tcatgaccag aactccctca caacccctct tctccctcgt 960

cctcgtcctc gcatctcacc tctccaacta cacatatact caaaaccctc aaccagaata 1020

ccaaccaaac cacccaaa 1038

<210> 32
<211> 1482
<212> DNA
<213> glaB cDNA

<400> 32
   atgcggaaca accttctttt ttccctcaat gccattgctg gcgctgtcgc gcatccgtcc 60
   ttccctatcc ataagaggca gtcggatctc aacgccttca ttgaggcaca gacacccatc 120
   gccaaacagg gcgtcctcaa taatatcggc gctgatggca agcttgttga gggggctgcc 180
   gctggtatcg ttgtagcctc cccatccaag agtaatcccg actacttcta cacctggacg 240
   cgcgacgctg gcctcaccat ggaagaagtg atagagcaat tcatcggggg agatgcgact 300
   ctcgagtcca caatccagaa ttatgttgac tctcaagcga acgagcaggc agtctccaac 360
   ccatcaggcg gcctgtcgga tggctcgggt cttgctgaac ccaaatttta cgtcaatatc 420
   tctcaattca ccgattcttg gggccgaccc cagcgcgacg ggccagcctt acgtgcttcc 480
   gctttgatcg catatggcaa ctctctgatt tccagcgaca aacaatctgt tgtcaaagct 540
   aacatctggc caattgtcca gaatgacttg tcttatgtgg gtcaatactg gaaccagacc 600
   gggtttgatc tttgggaaga ggttcagggc agctccttct tcactgttgc tgtgcagcac 660
   aaagccttgg tggagggcga tgcgtttgca aaggcactcg gagaggaatg ccaggcatgc 720
   tccgtggcgc ctcaaatcct ctgccatctt caggacttct ggaatgggtc tgctgttctt 780
   tctaacttac caaccaatgg gcgcagtgga ctggatacca actctctttt gggctccatt 840
   cacacttttg atccagccgc cgcttgtgat gatacaacat tccagccctg ctcctctcgc 900
   gccctgtcga accataagct tgtggttgac tctttccggt cggtctacgg tatcaacaat 960
   ggacgtggag caggaaaggc cgcggcagtg ggcccgtacg cagaggacac ctatcaggga 1020
   ggcaatccat ggtatcttac caccctggtc gctgcggaat tgctctacga cgccttgtat 1080
   cagtgggaca aacaaggtca agtgaacgtc actgaaactt cccttccctt cttcaaggac 1140
   ctctccagca atgtcaccac cggatcctac gccaagtctt cctcagccta tgagtcgctt 1200
   acgagcgctg tcaagaccta cgcagacggc ttcatctccg ttgtccagga gtatactccc 1260
   gatggcggtg ctttggctga gcagtacagt cgggaccagg gcaccccagt ttcggcatcc 1320
   gatctgactt ggtcttatgc agctttcttg agtgctgttg gacgacgaaa cggcactgtc 1380
   cctgctagct ggggctcttc cacggccaac gcagttccaa gccaatgttc ggggggtaca 1440
   gtttctggaa gttacactac cccaactgtt gggtcgtggt ag 1482

<210> 33
<211> 108
<212> DNA
<213> Artificial Sequence

<400> 33
tgctggatgg agtgggaccg caagatcgag gagtacacca agaagatcga ggagctcatc 60

aagaagtccc aggagcagca ggagaagaac gagaaggagc tcaagtgc 108

<210> 34
<211> 1038
<212> DNA
<213> glaB terminator

<400> 34
atgtactttc cagtgcgtgt agtctactct gacctcgtgt cacgattgtt gcttttgcct 60

gtctaaatgc gaccgtgctg tgcatgtttg ttaaatactg tcattcatct ttgtttcaac 120

aacaaagatt acatcaatta gtgctagcta gacaataact tttacagttg caacgttagt 180

cctagtatta tacatctcac cggatcctct tcaaacttca cggggtaacc aaaagaaagt 240

aacaagacta agcctattga tactgtggtt ctaatcttat tttagtttcc tgtacgtcca 300

ctgcaatcaa actaagtata catactacat cctagtatcc tacaccgtat ccttcaaccc 360

tagaacctcc cgctaggtat gtactgtaga tcgattatcc ggagattccc cgccacctgc 420

ttgaccaatc tgccgccctc agctgaaggc ccactggagt gaaaagacaa tggccgctcc 480

taacttcagc cgatatctaa cctcgacaat ccagcctcta ttcaaagaca gaaatctgtc 540

tacctcaaac tggctggtca ttgaccagaa gggtctggag atcggggttt gcttgttggc 600

gatcctgagg atatgtcgga tgagggggag tgtacgtttt tgattctttc tttctttctt 660

tctttctttt ctttgctatt tattttggat gctcgagtgg gagggaggta ttatttctct 720

ttagattgag ctccccatgt acaatgtata tatccgacat ggaacgggta tttttactac 780

ccacatagcc atgagctagc tatctttcaa cagtgttcga tgatggagtc tcgcttgacc 840

ataaccatat tctccagatt gcggttatcc aagactactg catatgcaga gtaattccag 900

cttataatag cggtcccaac caagatttga cattgaatca tatttccgcg ctcgtcagca 960

cagctttctt caacaaagca gaaggacatt gtctcagcat ttcgtccaga aggtatgtga 1020

aggtatagct ctgttcgc 1038

<210> 35
<211> 494
<212> PRT
<213> glaB

<220>
<221> MISC_FEATURE
<222> (494)..(494)
<223>

<400> 35

Met Arg Asn Asn Leu Leu Phe Ser Leu Asn Ala Ile Ala Gly Ala Val
1               5                   10                  15

Ala His Pro Ser Phe Pro Ile His Lys Arg Gln Ser Asp Leu Asn Ala
            20                  25                  30

Phe Ile Glu Ala Gln Thr Pro Ile Ala Lys Gln Gly Val Leu Asn Asn
        35                  40                  45

Ile Gly Ala Asp Gly Lys Leu Val Glu Gly Ala Ala Ala Gly Ile Val
    50                  55                  60

Val Ala Ser Pro Ser Lys Ser Asn Pro Asp Tyr Phe Tyr Thr Trp Thr
65                  70                  75                  80

Arg Asp Ala Gly Leu Thr Met Glu Glu Val Ile Glu Gln Phe Ile Gly

                85                  90                  95

Gly Asp Ala Thr Leu Glu Ser Thr Ile Gln Asn Tyr Val Asp Ser Gln
            100                 105                 110

Ala Asn Glu Gln Ala Val Ser Asn Pro Ser Gly Gly Leu Ser Asp Gly
        115                 120                 125

Ser Gly Leu Ala Glu Pro Lys Phe Tyr Val Asn Ile Ser Gln Phe Thr
    130                 135                 140

Asp Ser Trp Gly Arg Pro Gln Arg Asp Gly Pro Ala Leu Arg Ala Ser
145                 150                 155                 160

Ala Leu Ile Ala Tyr Gly Asn Ser Leu Ile Ser Ser Asp Lys Gln Ser
                165                 170                 175

Val Val Lys Ala Asn Ile Trp Pro Ile Val Gln Asn Asp Leu Ser Tyr
            180                 185                 190

Val Gly Gln Tyr Trp Asn Gln Thr Gly Phe Asp Leu Trp Glu Glu Val
        195                 200                 205

Gln Gly Ser Ser Phe Phe Thr Val Ala Val Gln His Lys Ala Leu Val
    210                 215                 220

Glu Gly Asp Ala Phe Ala Lys Ala Leu Gly Glu Glu Cys Gln Ala Cys
225                 230                 235                 240

Ser Val Ala Pro Gln Ile Leu Cys His Leu Gln Asp Phe Trp Asn Gly

                245                 250                 255

Ser Ala Val Leu Ser Asn Leu Pro Thr Asn Gly Arg Ser Gly Leu Asp
            260                 265                 270

Thr Asn Ser Leu Leu Gly Ser Ile His Thr Phe Asp Pro Ala Ala Ala
        275                 280                 285

Cys Asp Asp Thr Thr Phe Gln Pro Cys Ser Ser Arg Ala Leu Ser Asn
    290                 295                 300

His Lys Leu Val Val Asp Ser Phe Arg Ser Val Tyr Gly Ile Asn Asn
305                 310                 315                 320

Gly Arg Gly Ala Gly Lys Ala Ala Ala Val Gly Pro Tyr Ala Glu Asp
                325                 330                 335

Thr Tyr Gln Gly Gly Asn Pro Trp Tyr Leu Thr Thr Leu Val Ala Ala
            340                 345                 350

Glu Leu Leu Tyr Asp Ala Leu Tyr Gln Trp Asp Lys Gln Gly Gln Val
        355                 360                 365

Asn Val Thr Glu Thr Ser Leu Pro Phe Phe Lys Asp Leu Ser Ser Asn
    370                 375                 380

Val Thr Thr Gly Ser Tyr Ala Lys Ser Ser Ser Ala Tyr Glu Ser Leu
385                 390                 395                 400

Thr Ser Ala Val Lys Thr Tyr Ala Asp Gly Phe Ile Ser Val Val Gln

                405                 410                 415

Glu Tyr Thr Pro Asp Gly Gly Ala Leu Ala Glu Gln Tyr Ser Arg Asp
            420                 425                 430

Gln Gly Thr Pro Val Ser Ala Ser Asp Leu Thr Trp Ser Tyr Ala Ala
        435                 440                 445

Phe Leu Ser Ala Val Gly Arg Arg Asn Gly Thr Val Pro Ala Ser Trp
    450                 455                 460

Gly Ser Ser Thr Ala Asn Ala Val Pro Ser Gln Cys Ser Gly Gly Thr
465                 470                 475                 480

Val Ser Gly Ser Tyr Thr Thr Pro Thr Val Gly Ser Trp Xaa
                485                 490

<210> 36
<211> 36
<212> PRT
<213> Artificial Sequence

<400> 36

Cys Trp Met Glu Trp Asp Arg Lys Ile Glu Glu Tyr Thr Lys Lys Ile
1               5                   10                  15

Glu Glu Leu Ile Lys Lys Ser Gln Glu Gln Gln Glu Lys Asn Glu Lys
            20                  25                  30

Glu Leu Lys Cys
        35

<210> 37
<211> 30
<212> DNA
<213> Artificial Sequence

<400> 37
ttatgtactc cgtactcggt tgaattatta 30

<210> 38
<211> 30
<212> DNA
<213> Artificial Sequence

<400> 38
tgttccgcat tttgggtggt ttggttggta 30

<210> 39
<211> 30
<212> DNA
<213> Artificial Sequence

<400> 39
accacccaaa atgcggaaca accttctttt 30

<210> 40
<211> 30
<212> DNA
<213> Artificial Sequence

<400> 40
ccatccagca ccactgccgc ggcctttcct 30

<210> 41
<211> 30

<212> DNA
<213> Artificial Sequence

<400> 41
gcggcagtgg tgctggatgg agtgggaccg 30

<210> 42
<211> 30
<212> DNA
<213> Artificial Sequence

<400> 42
ttcacttgac gcacttgagc tccttctcgt 30

<210> 43
<211> 30
<212> DNA
<213> Artificial Sequence

<400> 43
gctcaagtgc gtcaagtgaa cgtcagtgaa 30

<210> 44
<211> 30
<212> DNA
<213> Artificial Sequence

<400> 44
gaaagtacat ctaccacgac ccaacagttg 30

<210> 45
<211> 30
<212> DNA
<213> Artificial Sequence

<400> 45

gtcgtggtag atgtactttc cagtgcgtgt                                    30

<210>  46
<211>  30
<212>  DNA
<213>  Artificial Sequence

<400>  46
gcgaacagag ctataccttc acataccttc                                    30

<210>  47
<211>  1197
<212>  DNA
<213>  glaB promoter

<220>
<221>  misc_feature
<222>  (422)..(422)
<223>  n stands for any base

<400>  47
tctcaaccca agtaacgatg aagaatggct gatcaaagcc agtttgcggg taactcattg      60

gccactaaca ccctgtgcag agagtatcta tagtagtaac aattgagtag taaagaaacg      120

cagtgtctgc aattggactt cgatatggaa aatatgtctc cgtccatggg catccggtcc      180

tacgcctccc atgtcaggag gtagtattct gcactggtat ggaaccctcc caagccaggc      240

tagctaaatt cactcctaga ctcattctta gagtagattc aaccagtcgt acttgcaagg      300

gtttcctgtg gaaaagaaca accagtcgaa gtggtggagg tcatgtgatg acgtcgaaga      360

gatggatgag tcataacgtc atgaactttg ctggtttctt caacttctcc acatctcact      420

tncaggtacc cgaggataat ccaaggagct ctatggctat agctgtacga gtgctacggc      480

atgccgtatt caactcaagc taacctaacc aaatacaaaa cggcagcaca tgccgatgca 540

taatgggagc tcaagacatt atcgtgtccg gccaaatggc ggcgaatcgg ccattcaccg 600

aacttggggc atctatctca aacgatgaga cctttacttg gcataggaaa gattgttaat 660

gggtgaagca tgctcctagt tgtatggccg atgtgcttcc ccgatcacaa ccgaaggttt 720

gtacatcact ccactccgcc ccgatcctgg cttaacctaa acacagtcaa gaataatagt 780

ccgccttcta agagcattaa gactcgacta ggactcagag gttcagtccg atggggtttc 840

caccagtgag aactaagaga atggcggcac gggcagatgt cgggatgatc acttttagtc 900

gctccgacgc aatatcgatt tcaattggat ccgccacgct gcatcccgga aattaatacc 960

ggatctgctt catgatgggg cctttggctg cggaaaactg ctgcgagtct cagtaatctc 1020

cgactgatcg gcccgctgcc tgttggttaa tgtcatgcag cccgttgcgg atcggttcac 1080

tgaattatct acgttacgtc taggtcgcgc tataaagaga cctgaaattc tcacttcgaa 1140

tgaggcagcc aacaaacagc tacagtcatt tcttgtttct tggaagtcac caccatc 1197

<210> 48
<211> 30
<212> DNA
<213> Artificial Sequence

<400> 48
tctcaaccca agtaacgatg aagaatggct 30

<210> 49
<211> 30
<212> DNA
<213> Artificial Sequence

<400> 49

tgttccgcat gatggtggtg acttccaaga 30

<210> 50
<211> 30
<212> DNA
<213> Artificial Sequence

<400> 50
caccaccatc atgcggaaca accttctttt 30